# EUROPÄISCHE PATENTSCHRIFT

(11) **EP 0 302 228 B1**
(45) Veröffentlichungstag und Bekanntmachung des Hinweises auf die Patenterteilung: **12.03.1997**
(21) Anmeldenummer: 88110465.7
(22) Anmeldetag: 30.06.1988
(51) Int. Cl.: G01N 27/333

(54) **Sensor zur Messung der Aktivität von Ionen und dessen Verwendung**
Probe for measuring the activity of ions and use thereof
Sonde pour la mesure d'activités d'ions et application de celle-ci

(30) Priorität: 01.08.1987 DE 3725597; 25.03.1988 DE 3810186
(43) Veröffentlichungstag der Anmeldung: 08.02.1989
(73) Patentinhaber: SIEGERT electronic GmbH, 90556 Cadolzburg (DE)
(72) Erfinder: Pfab, Werner, D-8502 Zirndorf (DE); Bergmann, Michael, D-8500 Nürnberg (DE); Fenzlein, Paul-Gerhard, D-8500 Nürnberg (DE); Anderer, Wolfgang, D-8521 Aurachtal (DE)

(56) Entgegenhaltungen:
- EP-A- 0 021 626
- EP-A- 0 061 810
- EP-A- 0 102 042
- EP-A- 0 115 346
- EP-A- 0 155 068
- EP-A- 0 186 286
- EP-A- 0 193 676
- EP-A- 0 204 260
- DD-A- 221 368
- FR-A- 1 560 716
- FR-A- 2 428 839
- FR-A- 2 445 525
- US-A- 4 370 983
- BIOMEDIZINISCHE TECHNIK, Band 22, Nr. 9, Seiten 209-211, 1977 J.G. SCHINDLER et al.: "Ionselektive Multi-Disk-Elektrode"

## Beschreibung

Die Erfindung betrifft zunächst einen Sensor zur Messung der Aktivität von Ionen mit Hilfe von ionenselektiven Membranen und zugehörigen Ableitungen. Eine derartige ionenselektive Membran besteht aus einer PVC-Grundstruktur, in welche entsprechend den zu messenden Ionen bestimmte Ionophore in bestimmten quantitativen Verhältnissen eingegeben werden. Derartige Ionophore sind chemische Verbindungen ganz bestimmter Struktur, die aus dem Stand der Technik bekannt sind. Je nach Anzahl der aktiven (freien) Ionen wird innerhalb der Membran und zur daran anliegenden Ableitung hin ein gewisses Phasengrenz-Potential gebildet, das man als elektromotorische Kraft (EMK) einer elektro-chemischen Halbzelle ansehen kann und ein Maß für die gemessene Ionenaktivität ist. Ionometrische Sensoren sind zur Durchführung von Serum- und Vollblutanalysen im Patientenbereich und im Labor bekannt. Der Artikel BIOMEDIZINISCHE TECHNIK, Band 22, Nr. 9, Seiten 209-211 (1977), beschreibt eine "ionenselektive Multi-Disk-Elektrode", deren mindestens eine Arbeitselektrode aus einer ionenselektiven Membran mit zu ihr in elektrischem Kontakt stehenden Ableitung aufgebaut ist und deren Bezugselektrode aus einer konventionellen Ag/AgCl-Bezugselektrode mit 3 M KCL Elektrolytlösung und PFTE-Membran besteht. In allen diesen Fällen wird das Blut, dessen Ionenaktivitäten gemessen werden sollen, als Probe (also invasiv) entnommen und in das Ionometer eingeführt, wobei es an einer Reihe von Membranen vorbei geleitet wird (Durchflußelektrode). Nachteilig ist hierbei, daß ein solches Ionometer nur für einen ganz bestimmten, vorstehend geschilderten Anwendungszweck geeignet ist und daß bei jeder Messung eine bestimmte Menge Vollblut (mindestens 2 bis 3 ml) dem Patienten entzogen werden muß. Dies ist für anämische (blutarme) (Dialyse-) -Patienten und Kinder besonders belastend. Darüber hinaus besteht für das Personal bei jeder Blutabnahme prinzipiell eine akute Ansteckungsgefahr (besonders Hepatitis B und AIDS).

Die Aufgabe der Erfindung besteht zunächst demgegenüber darin, einen Sensor zur Messung der Aktivität von Ionen mit Hilfe von ionenselektiven Membranen und zugehörigen Ableitungen, dahingehend auszugestalten, daß mehrere Membranen und damit mehrere gleichzeitige Möglichkeiten zur nichtinvasiven Messung unterschiedlicher oder gleicher Ionen für den gesamten medizinischen (therapeutischen und diagnostischen) Bereich, aber auch an Flüssigkeiten zur Verfügung stehen, wobei die Möglichkeit bestehen soll, einen solchen Sensor auf sehr kleinem Raum unterzubringen.

Die Lösung dieser Aufgabe wird daher ausgehend von einem Sensor gemäß dem genannten Oberbegriff des Anspruches 1 zunächst darin gesehen, daß zur gleichzeitigen Messung mehrerer Ionenaktivitäten an der Oberfläche von organischen Geweben oder in Flüssigkeiten zumindest zwei ionenselektive Membranen in einem gemeinsamen isolierenden Träger gehalten sind, daß jeder dieser Membranen eine Ableitung zugeordnet ist und zu ihr in elektrischem Kontakt steht, daß ferner jede Membran im Träger sowohl elektrisch isoliert, als auch gegen den Durchtritt von Feuchtigkeit gesichert gehalten ist, daß entweder eine der jeweils aus einer Membran und einer Ableitung bestehenden Elektroden als Bezugselektrode ausgebildet ist, oder daß eine Bezugselektrode als gesonderter Bauteil mit zugehöriger Kontaktmöglichkeit an das organische Gewebe, bzw. die Flüssigkeit vorgesehen ist, während die übrigen Elektroden als Meßelektroden dienen, daß zumindest alle Meßelektroden in einem Sensorkörper zusammengefaßt sind und daß der Träger und der Sensorkörper aneinander fest gehalten sind. Menschliche und tierische Organe und Gewebe sind in vivo von einem dünnen Flüssigkeitsfilm aus interstitieller Flüssigkeit überzogen. Die in ihm enthaltenen Ionen sin ein gutes Maß für die Funktion des jeweiligen Organs und können mit der Erfindung gemessen werden. Weiterhin läßt sich dadurch sogar über eine räumliche Verteilung der einzelnen Ionenaktivitäten ein Rückschluß über die Funktion kleinster Gebiete, bis hin auf die Ebene der Kapillaren (Mikrozirkulation) ziehen. Bei Anwendung der Erfindung müssen weder Proben genommen werden, noch kommt es zu irgendeiner Schädigung des zu messenden Gewebes. Während der Dialyse (siehe unten) sind kontinuierliche Messungen direkt im Blut des Patienten ohne die Belastung durch Blutabnahmen möglich. Direkte Blutmessungen ermöglichen ein "on-line monitoring" ohne zeitliche Verzögerungen. Bei Messungen von Ionen unterscheidet man Ionenkonzentrationen und Ionenaktivitäten. Ionenkonzentration ist die Gesamtheit der einzelnen Ionen (z.B. alle Natriumionen), die im Körper oder im Blut gemessen werden können. Bei der Ionenaktivität werden nur die freien Ionen gemessen, welche nicht an andere Substanzen gebunden sind, und somit aktiv an den Stoffwechselfunktionen der Organe teilnehmen können. Nur diese aktiven Ionen können von Ionophoren eingefangen werden, wodurch eine Änderung des oben genannten Phasengrenz-Potentials an der messenden Elektrode entsteht. Die Anwendungsbereiche der Erfindung liegen daher zunächst und bevorzugt im medizinischen Bereich, wie: Transplantationszentren, chirurgischen und intensivmedizinischen Abteilungen und in der allgemeinen Medizintechnik. Ferner ist der Einsatz in der oben genannten Dialysebehandlung und der medizinisch-pharmazeutischen Forschung möglich. Es können damit zurzeit bestehende Risiken in medizinischen Behandlungen durch schnelle und gleichzeitige Gewinnung wichtiger Informationen über den Elektrolyt-Haushalt einzelner Organe (z.B. Herz, Niere, Gehirn u.a.), in der Chirurgie (z.B. der Transplantationschirurgie) und den weiteren angegebenen medizinischen Bereichen deutlich verringert werden. Wie oben erklärt ist die Messung der biologisch relevanten Aktivitäten der unterschiedlichen Ionen möglich, aber auch Ionenmessungen in nicht medizinischen Bereichen.

Ferner kann dieser direkt kontaktierende, ionenselektive Mehrfach-Oberflächensensor mit seinem Sensorkörper, Träger und Membranen und den Membranen zugeordneten Ableitungen eine bewegliche Einheit bildet, die lediglich über ein Verbindungskabel mit einem Rechner oder dergleichen (rechnergesteuerte Meßdatenverarbeitung mit normierten, digitalen Schnittstellen) verbunden, aber demgegenüber beweglich ist und daher z.B. an jeder gewünschten Stelle eines Organes zwecks Durchführung der Messung aufgesetzt werden kann. Dabei ist ein Organ nicht nur eines der vorstehend beispielsweise erwähnten Organe wie Herz, Niere, oder Gehirn, sondern auch die Haut eines Patienten, auf deren Oberfläche der Sensor aufgesetzt wird und die erforderlichen Messungen vornimmt. Die vorstehend erläuterte Beweglichkeit, bzw. Portabilität der Messanordnung ist sowohl im medizinischen, im biomedizinischen, oder pharmazeutischen Anwendungsbereich für den Einsatz dieses Sensors in der Praxis von großem Vorteil. Der oben genannte Begriff der Portabilität schließt insbesondere auch ein, daß ein solcher Sensor mit zugehöriger Elektronik relativ klein und beweglich ist, z.B. in einem Hubschrauber mitnehmbar oder in einem anderen Beispiel problemlos von einem Operationssaal in einen anderen Raum transportiert werden kann.

Im übrigen ist für die elektrische Isolierung jeder der Membrane, für einen Schutz gegen den Durchtritt von Feuchtigkeit und für die Zusammenfassung aller Meßelektroden an bzw. in einen Sensorkörper gesorgt. Bevorzugt ist die ferner vorgesehene Bezugselektrode eine der im oder am Sensorkörper gehaltenen Elektroden, da dies die wirtschaftlich billigste und günstigste, sowie hinsichtlich der Raumbeanspruchung auch kleinste Ausführung ist. In Sonderfällen, bzw. wenn erwünscht, kann aber auch eine gesonderte Bezugselektrode wie angegeben vorgesehen sein. Zu Vorstehendem wird nochmals betont, daß hier mit dem Begriff der "Elektrode" die Einheit aus einer Membran und einer dazu gehörigen Ableitung zu verstehen ist. Der Träger schützt die Membranen gegen mechanische Beschädigungen.

Der Vollständigkeit halber sei erwähnt, daß man aus DE-OS 29 27 948 einen hohlzylindrischen Körper kennt, in dessen Inneren ein Kolben dicht gleitend verschiebbar ist. Die Stirnfläche dieses Kolbens ist mit Metallelektroden oder Glaselektroden versehen, die vom Kolbenkopf vorstehen und Messungen an einer Flüssigkeit vornehmen, die in den Kolben einzubringen ist. Wenn auch Glaselektroden ionenselektiv sind, so kann man damit nur in Flüssigkeiten messen, nicht aber gemäß der Erfindung auf einer Organoberfläche. Ein weiterer wesentlicher Nachteil der Anordnung nach DE-OS 29 27 048 und eine ähnliche Anordnung nach DE-OS 29 24 117 besteht darin, daß diese Elektroden ein Vielfaches des Raumes bzw. der Kontaktfläche benötigen, der mit einem Sensor und Membrananordnung nach der Erfindung beansprucht wird. Dieser Unterschied kann mehr als eine Zehnerpotenz ausmachen. Schließlich erfüllen die zuletzt genannten Literaturstellen nicht den eingangs zitierten Oberbegriff des Anspruches 1 und zeigen auch nicht die vorstehend angegebenen Merkmale und Vorteile der Erfindung.

Nach der Erfindung können alle Membrane eines Trägers auf ein bestimmtes Ion eingestellt sein. Dies ergibt die Möglichkeit, die Verteilung der Aktivitäten eines gleichen Ions über eine bestimmte Körperoberfläche zu messen und numerisch oder grafisch darzustellen. Dagegen ist es mit der Erfindung aber auch möglich, daß alle Membrane eines Trägers jeweils auf unterschiedliche Ionen eingestellt sind. Hierdurch ist erreichbar, mit ein- und demselben Sensor gleichzeitig die Aktivitäten unterschiedlicher Ionen festzustellen. Es sind aber nach der Erfindung auch Messungen der Kombinationen der beiden vorgenannten Möglichkeiten denkbar.

Die bereits erwähnte Beweglichkeit des Sensors kann gemäß einer bevorzugten Ausführungsform der Erfindung mittels einer Haltevorrichtung geschehen, an der der Sensor angebracht ist. Dabei ist die Haltevorrichtung entweder als Handgriff ausgebildet oder mit einem Handgriff versehen. Dies vereinfacht und erweitert den Einsatz des Sensors nach der Erfindung in der Praxis wesentlich. Hierbei ist es ein besonderer Vorteil, wenn der Sensor leicht gegen einen Sensor mit anderen Membranen austauschbar ist. Bei Vorhandensein einer Haltevorrichtung ist der jeweilige Sensor daran lösbar zu befestigen, wobei eine lösbare Kontaktierung zwischen den Ableitungen des Sensors und entsprechenden Anschlüssen der Haltevorrichtung besteht. Die Membrane der jeweils anderen Sensoren können nach Art und Anzahl von denen des vorhergehenden Sensors verschieden sein. Damit ist die Möglichkeit gegeben, sich an verschiedene spezielle Anforderungen anpassen zu können.

Es kann eine Rechnerelektronik vorgesehen und zumindest teilweise in der Haltevorrichtung untergebracht sein. Das gleiche gilt für einen Impedanzwandler, der funktionell zwischen dem jeweiligen Sensor und der Rechnerelektronik vorgesehen ist. Auch der nicht im Handgriff untergebrachte Teil der Rechnerelektronik kann tragbar sein. Ein solches Meßsystem (Sensor-Messung von Ionenaktivitäten; Sensor-Halter-Impedanzwandlung und Signalverstärker; Analog-Digital-Wandlung zum Rechner und Meßdatenverarbeitung) kann nicht nur stationär eingesetzt sondern auch als transportables Meßsystem (einschließlich der Transportmöglichkeit mittels der Haltevorrichtung) aufgrund seiner Beweglichkeit, bzw. Portabilität wie erläutert verwendet werden.

Eine bevorzugte Ausführung der Erfindung besteht darin, daß die Membrane räumlich dicht beieinander auf einer möglichst kleinen Fläche angeordnet sind, z.B. in Form von Kreisen, einer Spirale, einer Linie oder einer sogenannten Matrix. Dies ermöglicht, mit einem kompakten und daher gut zu handhabenden und mittels einer großen Anzahl gleicher oder unterschiedlicher ionenselektiver Membranen an die Organoberfläche zu legenden Sensor auf sehr kleiner Fläche messen zu können.

Um die vorgesehene Anzahl von Ableitungen isoliert auf kleinem Raum unterzubringen kann nach einer bevorzugten Ausführungsform der Erfindung ein Sensorkörper aus einer Reihe von miteinander fest verbundenen Schichten vorgesehen sein. Diese einzelnen Schichten weisen voneinander und nach außen isoliert leitende Strukturen als Ableitungen auf, welche an einer Stirnfläche des Sensorkörpers Kontaktstirnflächen bilden. Jede dieser Ableitungen ist mit einem zugehörigen Anschlußkontakt oder einer Anschlußfläche des Sensorkörpers, bevorzugt auf dessen obersten Schicht, elektrisch leitend verbunden. Bevorzugt ist hierzu ein Multilayersubstrat vorgesehen, das aus schichtweise übereinander liegenden und miteinander verbundenen, mit den Ableitungen versehenen Keramikfolien oder polymeren Folien besteht.

Der Träger der Membrane kann gemäß einer Ausführung der Erfindung ein gesondertes Teil sein, das auf einer von dem jeweiligen Sensorkörper gebildeten Stirnfläche aufliegt und daran befestigt ist. Dabei befinden sich die Kontaktflächen der Ableitungen in den Stirnflächen des Sensorkörpers und stehen in Kontakt mit den entsprechenden Gegenflächen der Membrane (siehe hierzu die obigen Ausführungen zum Phasengrenz-Potential).

Als Alternative zu der letztgenannten Ausführungsform der Erfindung können Träger und Sensorkörper auch einstückig sein. Hierzu können als Ableitungen die Enden voneinander isolierter und zu einem Bündel zusammengefaßter Drähte dienen. Dabei sind diese Bündel durch eine Vergußmasse zusammengehalten, die zugleich den Sensorkörper und den Träger für die Membranen bildet. Ein solcher Sensor kann sehr langgestreckt und dabei im Durchmesser sehr klein (kleiner als bei der zuvor erläuterten Ausführungsform) gebaut werden. Er eignet sich daher besonders als Sonde zum Einbringen in einen menschlichen Körper, wobei er mehrere Tage im Körper verbleiben kann, um Messungen durchzuführen. Anschließend kann diese Sonde herausgezogen werden. Die vorgenannten Messungen können z.B. Messungen an der Oberfläche eines Organes sein.

In beiden vorgenannten Fällen ist ein einwandfreier elektrischer Kontakt im Sinn der Erfindung zwischen Ableitungen und zugehörigen Membranen ermöglicht.

Es ist bereits der Einsatz eines solchen Sensors für die Hämodialyse genannt worden. Hierzu können nach einem weiteren Vorschlag der Erfindung Sensoren und eine zugehörige Anordnung, zumindest zwei Sensoren vorgesehen sein, von denen der eine (erste) Sensor in einem vom Blut des Patienten durchflossenen Kreislauf an einer dem Dialysator in Richtung dieses Kreislaufes nachgeordneten Stelle und der andere (zweite) Sensor im Verlauf der Dialyseflüssigkeit in deren Fließrichtung an einer vor dem Dialysator gelegenen Stelle zwecks ionenselektiver Messung des Blutes und der Dialyseflüssigkeit eingeschaltet sind, wobei die Meßergebnisse beider Sensoren zwecks Regelung der Zusammensetzung der Dialyseflüssigkeit einer Regeleinrichtung zugeführt werden und der Regeleinrichtung ein Vergleichs-Sollwert des gereinigten Blutes zur Verfügung steht. Diese vorteilhafte ionenselektive Messung, die bevorzugt temperaturkompensiert erfolgt, ist somit als Hämodialyse-Messvorrichtung zur Steuerung des Dialyseflüssigkeitsmischsystemes ausgebildet, welche zwei Kreisläufe, nämlich den Blutkreislauf und den Kreislauf der Dialyseflüssigkeit, in ihrem Gehalt an bestimmten chemischen Bestandteilen, z.B. Kalium, mißt. Die jeweiligen Gehalte an bestimmten chemischen Bestandteilen müssen in beiden Kreisläufen aufeinander abgestimmt werden, damit im Dialysator (sogenannte "künstliche Niere") die jeweils erforderliche Menge an diesen chemischen Bestandteilen vom Blutkreislauf in den Kreislauf der Dialyseflüssigkeit übergeht; gegebenenfalls auch umgekehrt. Die hierfür und insbesondere für die Einstellung der chemischen Bestandteile der Dialyseflüssigkeit notwendigen Meßdaten werden von den Sensoren gemäß der Erfindung geliefert und der zugehörigen Regeleinrichtung zugeführt. Die Anordnung nach der Erfindung arbeitet "on line" und zwar während des Dialysevorganges. Zur Messung benutztes Blut muß nicht verworfen werden.

In einer bevorzugten Ausführungsform der Erfindung ist für die ionenselektive Messung des Blutes ein weiterer (dritter) Sensor vorgesehen, der sich in Richtung des Kreislaufes des Blutes vor dem Dialysator befindet, dessen Meßdaten ebenfalls der Regeleinrichtung zugeführt werden. Dieser (dritte) Sensor wirkt mit dem genannten (ersten) Sensor dahingehend zusammen, daß dadurch die "Reinigung" des Blutes überwacht und eine schlechte "Reinigung" erkannt wird. Auch könnte unter Umständen eine den Patienten belastende oder sogar gefährdende zu schnelle "Reinigung" eintreten. Beide vorgenannten Fälle können durch das Zusammenwirken dieses dritten Sensors mit den davor erläuterten ersten und zweiten Sensoren erkannt und der Regeleinrichtung und/oder dem Überwachungspersonal gemeldet werden.

Schließlich kann gemäß einem weiteren Vorschlag der Erfindung in den Verlauf der Dialyseflüssigkeit ein weiterer, vierter Sensor eingeschaltet sein, der sich in der Fließrichtung der Dialyseflüssigkeit betrachtet nach dem Dialysator befindet und dessen Meßdaten ebenfalls der Regeleinrichtung zugeführt werden. Das Zusammenwirken dieses vierten Sensors mit dem oben genannten zweiten Sensor ermöglicht eine entsprechende Gegensteuerung, wobei der zweite Sensor zur Kontrolle des Dialysatmischsystemes dient und der vierte Sensor zur qantitativen Kontrolle des Elektrolytverlustes während der Dialyse.

Dialyseeinrichtungen bestehen vom Prinzip her aus einem Dialysator ("künstliche Niere"), einem die Blutzusammensetzung überwachenden Blutmonitor und einem die Dialysatzusammensetzung überwachenden Dialysatmonitor. Hierzu ist von Vorteil, wenn die Sensoren mit ihren Sensorkörpern jeweils in einer Meßzelle untergebracht sind, um problemlos die jeweiligen Anschlüsse herzustellen, bzw. bei Erfordernis wieder lösen zu können. Eine solche Meßzelle kann an den vorgenannten Monitoren befestigt werden.

Die vorgenannten Meßzellen haben Anschlußstellen. Dabei kann gemäß einer weiteren Ausführungsform der Erfindung eine weitere Anschlußstelle für den Anschluß und Zufuhr einer Eichflüssigkiet und ein Kanal von dieser Anschlußstelle zu einem Dreiwegehahn vorgesehen sein. Vom Dreiwegehahn führt ein weiterer Kanal zur Anschlußstelle für die Flüssigkeitszufuhr. Der Dreiwegehahn ist wahlweise in zwei Stellungen bringbar. In der einen Stellung dient er zur Verbindung der Anschlußstelle für die Eichflüssigkeit mit dem Sensor und in der anderen Stellung für eine Verbindung der Anschlußstelle für die Flüssigkeitszufuhr mit dem Sensor. Dies ermöglicht mit derartigen Meßzellen die Vornahme einer Eichung oder sogenannten Kalibrierung, wobei mittels des Dreiwegehahnes ohne Unterbrechung und ohne Lösung von Verbindungen bzw. Anschlüssen von der Messung der jeweiligen Flüssigkeit auf die Eichung übergegangen werden kann und umgekehrt. Es sind also Zwischenkalibrierungen möglich. Dabei ist es lediglich erforderlich, daß die Eich- oder Kalibrierflüssigkeit nach der Kalibrierung der jeweils zu messenden Flüssigkeit (Blut oder Dialyseflüssigkeit) zugegeben werden kann. Die bereits erwähnte "on line" Messung, die kontinuierlich erfolgen kann, kann ebenso kontinuierlich als Eichmessung durchgeführt werden. Man kann ohne Zeitverlust wieder in die eigentliche Flüssigkeitsmessung übergehen. Auch in diesem Falle sind gesonderte Blutabnahmen oder Blutverluste nicht erforderlich, bzw. nicht gegeben.

Ein Temperaturfühler, der in den Sensor eingebaut ist, ist bei der Dialyse besonders von Vorteil, um der Regeleinrichtung bzw. dem Rechner die notwendige Temperaturkorrektur zu liefern. Man erhält also eine ionenselektive, temperaturkorrigierte Dialysevorrichtung mit Dialysatmischsystem zur exakten Anpassung der im Dialysat enthaltenen Elektrolyte an die besonderen Bedingungen, bzw. Blutzusammensetzung des jeweiligen Patienten.

Eine weitere vorteilhafte Ausgestaltung der Erfindung hinsichtlich der Membrane und ihrer Anordnung im Träger besteht darin, daß zwei oder mehrere unterschiedliche Membrane in einem Träger übereinander vorgesehen sind und daß jede der Membrane mit der benachbarten Membrane in Berührungskontakt steht. Die vorgenannten Membrane bilden also übereinander angeordnet eine "Säule". Es versteht sich, daß gemäß dem eingangs erläuterten Prinzip der Erfindung mehrere dieser "Säulen" in einem gemeinsamen Träger vorgesehen sind, wobei dieser Träger mit einem Sensorkörper zusammengesetzt ist, bzw. dieser Träger und der Sensorkörper die einstückige Einheit sein können. Dabei wandert die zu messende Flüssigkeit von der äußeren Kontaktfläche her durch die einzelnen Membrane hindurch. Man hat mit dieser Anordnung die Möglichkeit, in der zu messenden Substanz bzw. Flüssigkeit vorhandene Störionen durch eine der Membrane binden, bzw. absorbieren zu können. Dies erhöht den Genauigkeitsgrad der Messung des jeweiligen Ions, wobei aber nicht mehr an Grundfläche benötigt wird, als bei den Ausführungsbeispielen, bei denen in jedem Durchbruch oder Vertiefung des Trägers nur eine Membran vorgesehen ist.

Ein weiteres Ziel der Erfindung besteht in der Schaffung von Verfahrensschritten zur Herstellung des Sensorkörpers mit Ableitungen und/oder des Trägers mit Membranen. So kann z.B. die oben beschriebene Ausführungsform, bei der Träger und Sensorkörper einstückig aus Vergußmasse sind, in der Weise hergestellt werden, daß zunächst die Drähte des Bündels miteinander verbunden und danach mit der Vergußmasse vergossen werden, daß danach entsprechend der Dicke der Membranen die Metallseelen der Drähte von der den Träger bildenden Stirnseite der Vergußmasse her entfernt, z.B. weggeätzt werden und daß danach die Membrane in die somit gebildeten Vertiefungen eingebracht werden.

Weitere Vorteile und Merkmale der Erfindung sind der nachstehenden Beschreibung und der zugehörigen Zeichnung von erfindungsgemäßen Ausführungsmöglichkeiten zu entnehmen. Generell wird in dem Zusammenhang auf den Inhalt der Ansprüche verwiesen, die vorstehend bereits im wesentlichen angeführt und im übrigen auch in der Beschreibung erläutert sind. In der Zeichnung zeigt:
- Fig. 1:: eine prinzipielle Darstellung einer ersten Ausführungsform der Erfindung im Längsschnitt,
- Fig. 2:: eine weitere, prinzipielle Darstellung einer anderen Ausführungsmöglichkeit der Erfindung,
- Fig. 3:: die einzelnen Schichten eines Multilayersubstrates nach der Erfindung in Explosionsdarstellung, jedoch ohne Träger und Membranen,
- Fig. 4:: eine Stirnansicht in Richtung des Pfeiles IV auf einen Sensorkörper gemäß Fig. 3,
- Fig. 5:: eine Ansicht analog Fig. 4, jedoch mit den Membranen und deren Träger,
- Fig. 6:: eine Draufsicht gemäß der Pfeilrichtung VI in Fig. 5,
- Fig. 7:: ein weiteres Ausführungsbeispiel der Erfindung in der Draufsicht,
- Fig. 8:: einen Schnitt gemäß der Linie VIII-VIII in Fig. 7,
- Fig. 9:: in einer vereinfachten, perspektivischen Darstellung eine bewegliche, portable Anordnung nach der Erfindung,
- Fig. 10:: eine schematische Darstellung einer Dialyseanordnung mit zugehörigen Sensoren nach der Erfindung,
- Fig. 11:: einen Schnitt durch eine Meßzelle nach der Erfindung gemäß der Linie XI - XI in Fig. 12,
- Fig. 12:: eine Ansicht gemäß dem Pfeil XII in Fig. 11,
- Fig. 13:: einen Schnitt gemäß der Linie XIII -XIII in Fig. 11,
- Fig. 14:: schematisch und im Schnitt die Ausbildung eines Sensors nach der Erfindung mit mehreren übereinander befindlichen Membranen,
- Fig. 15:: eine weitere Variante der Ausbildung von Sensoren nach der Erfindung mit mehreren übereinander angeordneten Membranen.

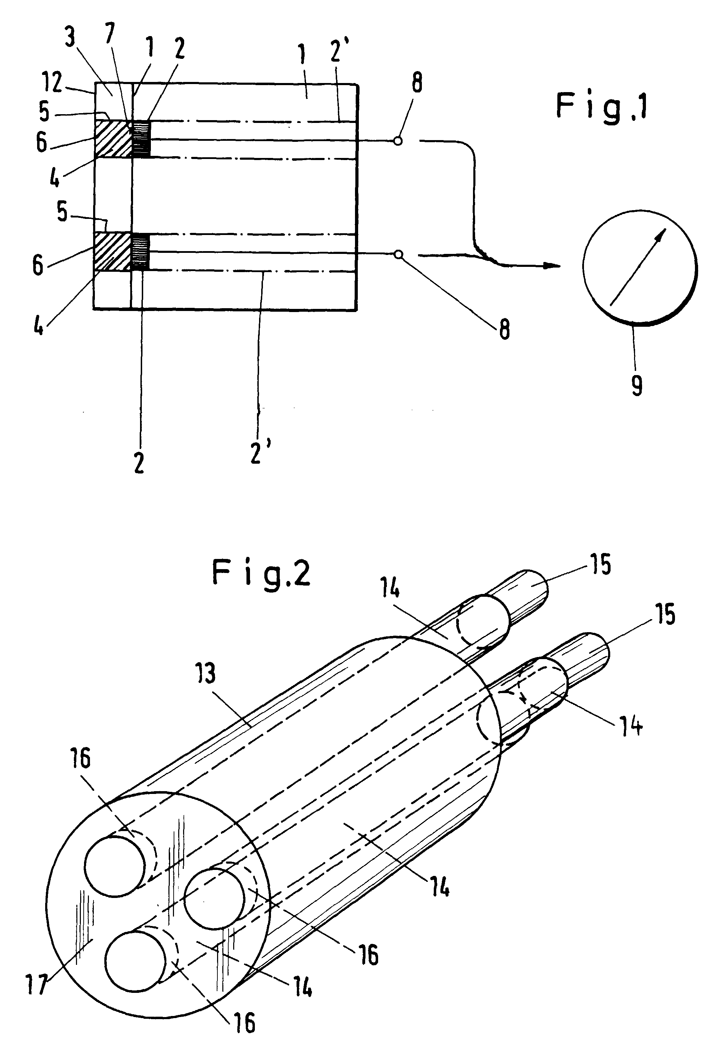

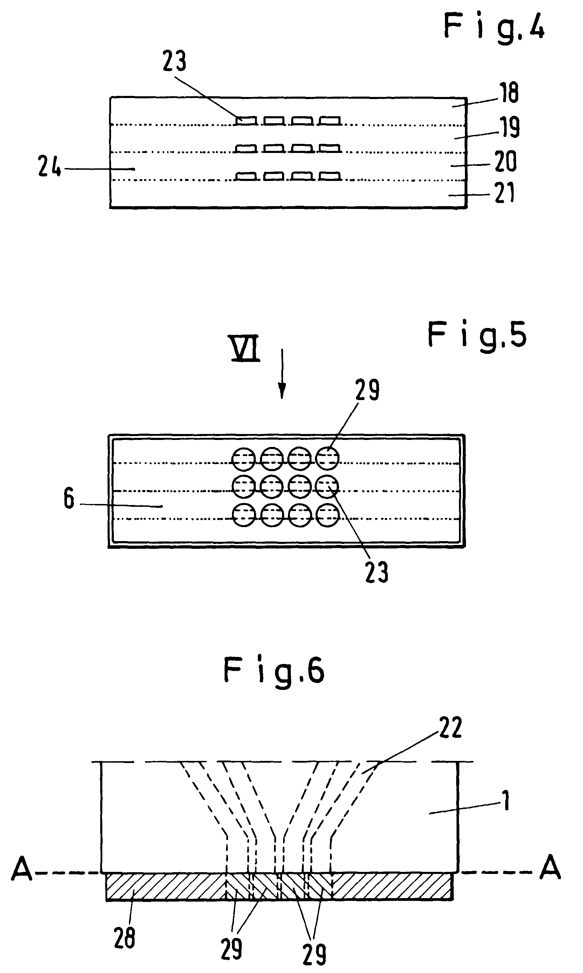

Fig. 1 zeigt den prinzipiellen Aufbau der Erfindung mit einem Sensorkörper 1, der die Ableitungen 2 aufnimmt und hält, sowie einem Träger 3 für Aufnahme und Halt der Membrane 4. Jede Membrane mit der zugehörigen Ableitung bildet eine als Elektrode definierte Einheit. Der Träger 3 liegt auf der Stirnfläche 1' des Sensorkörpers 1 auf und ist daran fest gehalten. Es sind in diesem Beispiel zwei Membranen 4 vorgesehen. Durch das Material des Trägers 3, bevorzugt ein Polymer, sind die Membranen 4 voneinander elektrisch isoliert und ist zugleich dafür gesorgt, daß an den Berührungsflächen 5 keine Spalten zwischen Membranmaterial einerseits und Trägermaterial andererseits entstehen können, durch die Feuchtigkeit eindringen oder sogar hindurchtreten könnte. Damit sind parasitäre Effekte verhindert. Durch solche Spalten würde es nämlich zu einem Kurzschluß zwischen der äußeren Kontaktfläche 6, die zur Anlage an eine Körperoberfläche oder dergleichen bestimmt ist, einerseits und der inneren Kontaktfläche 7 der Membran mit der jeweiligen Ableitung andererseits führen. Damit wäre es aber nicht mehr möglich, selektiv das Phasengranz-Potential für das entsprechende Ion zu messen. Das angestrebte Meßergebnis könnte also im Falle eines Feuchtigkeitsein- oder oder-durchtrittes in den Spalt 5 nicht erreicht, zumindest sehr verfälscht werden. Der Querschnitt der Membranen kann unterschiedlich sein, bevorzugt hat er die in den Fig. 2 und 5 dargestellte Kreisform, die sich auch zur Erzielung der erläuterten Feuchtigkeitsdichtigkeit empfiehlt.

Die im elektrischen Berührungskontakt mit den Membranen 4 stehenden Ableitungen 2 können, wie es mit Ziffer 2' strichpunktiert angedeutet ist, durch den gesamten Sensorkörper 1 hindurchgeführt werden. Die Ableitungen 2 enden in Anschlüssen 8, die zu einer nur angedeuteten Rechnerelektronik 9 oder einer sonstigen Meß- oder Verarbeitungseinrichtung geführt werden können.

Es ist zumindest eine der o.g. Elektroden 4, 2 eine Meßelektrode. Die andere Elektrode hat die Funktion einer Bezugselektrode. Die Bezugselektrode wird an die Organoberfläche oder die zu messende Flüssigkeit angelegt. Die Bezugselektrode besteht ebenso wie die Meßelektroden aus einer entsprechenden Membran mit Ableitung.

Die jeweilige Bezugselektrode bildet einen konstanten Bezugspunkt für die Meßelektrode, bzw. die Meßelektroden des Sensors zwecks Messung der Spannungsdifferenzen zwischen ihr und den einzelnen Meßelektroden. Es sind also die Ableitungen und Membranen des ionenselektiven Sensors die eine Hälfte eines Meßkreises, dessen andere Hälfte von der Bezugselektrode gebildet wird.

Jede Einheit aus Membran und Ableitung, also jede Elektrode, ist von den anderen Elektroden elektrisch isoliert. Die Elektroden sind in Gruppen am oder im jeweiligen Sensorkörper zusammengefaßt, der leicht an der jeweiligen Haltevorrichtung austauschbar ist. Dies wird nachstehend noch näher erläutert.

Die zum Kontakt mit der Organoberfläche oder Flüssigkeit bestimmten Außenflächen 6 der Membranen 4 sind mit der Außenfläche 12 des Trägers bündig.

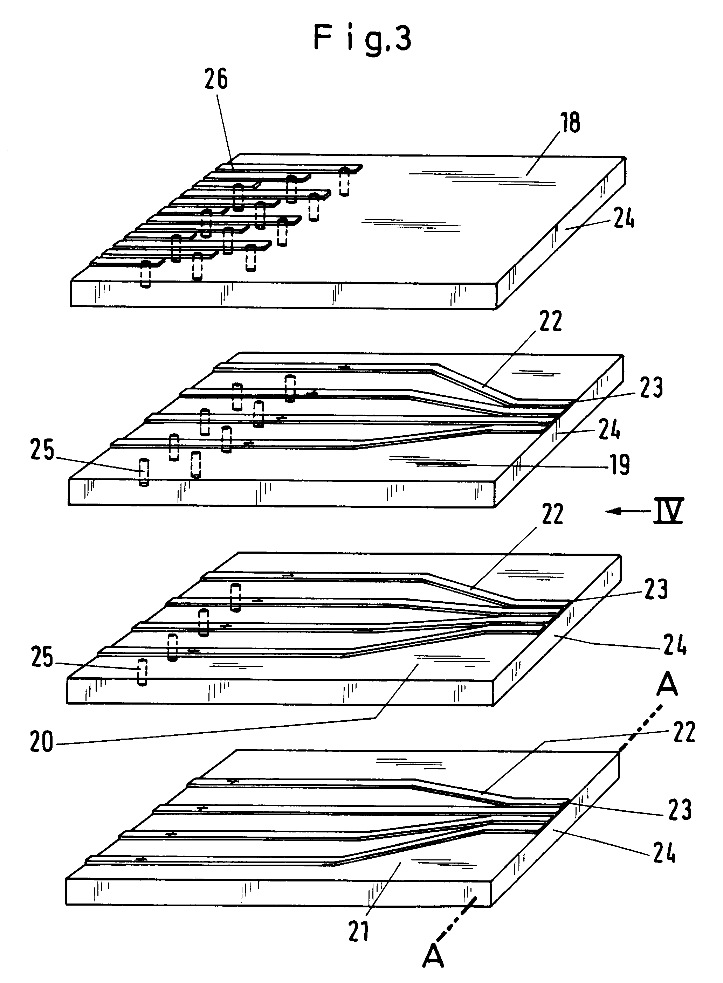

Fig. 2 zeigt eine andere Ausführung der Erfindung, bei der der Sensorkörper 1 und der Träger 3 ein in sich einheitlicher Teil 13 aus einer Vergußmasse, z.B. einem Polymer, sind. In der Vergußmasse befinden sich die als Drähte ausgebildeten Ableitungen 14 mit isolierten Anschlüssen 15, sowie die Membranen 16, welche in die in Fig. 2 links gezeichnete Stirnfläche 17 der sie isolierenden Vergußmasse eingelassen sind. Es können zunächst die Ableitungen 14 mit Vergußmasse 13 im Strang hergestellt werden, z.B. durch Bündeln der Ableitungen 14 und Umgießen mit der Vergußmasse. Danach kann man auf der Stirnfläche 17 durch Abätzen der Ableitungen Vertiefungen schaffen, in denen die Membranen 16 untergebracht werden. Hinsichtlich Einzelheiten der Befestigung und feuchtigkeitsdichten Anordnung der Membranen innerhalb des Trägers (dies gilt auch für Fig. 1) wird auf die späteren Erläuterungen zu den Fig. 3 bis 6 verwiesen.

Es ist ersichtlich, daß somit an der Oberfläche des Organes eines Körpers, d.h. nicht invasiv, die momentane Ionenaktivität gemessen werden kann mit der Möglichkeit, damit auf die Mikrozirkulation in Geweben und Organen rückzuschließen. Mit dem Sensor kann sowohl bei Vorhandensein gleichartiger Membranen zugleich eine große Anzahl gleichartiger Ionen als auch in einer anderen Ausführung mit unterschiedlichen Membranen gleichzeitig unterschiedliche Ionen, z.B. H⁺, K⁺, Na⁺, Ca²⁺ o.a. jeweils auf einem sehr kleinen Raum gemessen werden. Es ist die flächige Verteilung der Ionenaktivitäten entsprechend der jeweiligen räumlichen Anordnung der Membranen gleichzeitig mess- und darstellbar. Die vorgenannten Meßmöglichkeiten können auch an einem Sensor kombiniert vorgesehen sein.

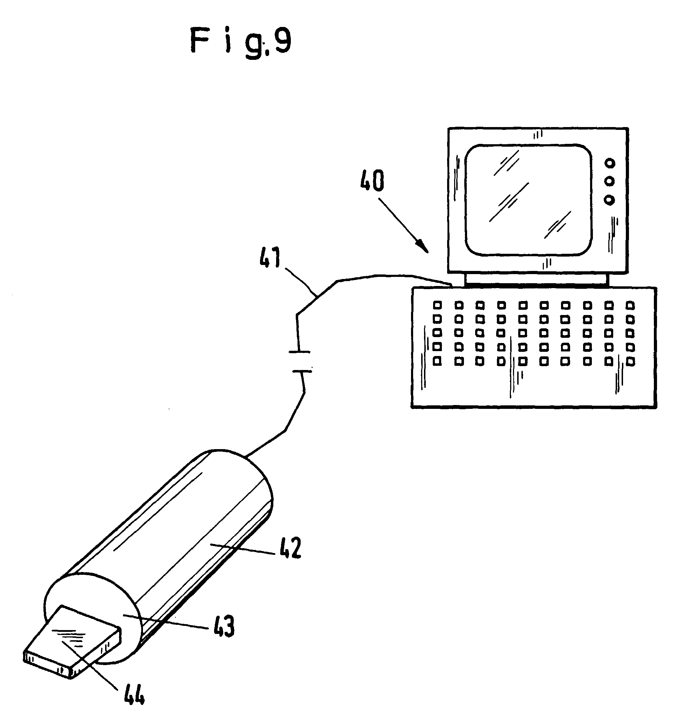

Das Ausführungsbeispiel der Fig. 3 bis 6 zeigt die Zusammensetzung eines Sensors, bzw. Sensorkörpers aus mehreren Schichten, die miteinander laminiert sind, zu einem sogenannten Multilayersubstrat. Unter Laminieren wird eine Technik verstanden, welche solche Schichten unter Druck und Temperatur zusammenbringt und aneinander "backt", so daß sie innig miteinander verbunden sind. Die hier mit 18 bis 21 bezifferten vier Schichten können z.B. jeweils eine Keramikfolie oder eine polymere Folie sein. Anschließend werden die Folien noch im Ofen gebrannt oder gehärtet. Diese Technik ist bekannt. Von den hier dargestellten vier isolierenden Substratschichten 18 bis 21 sind die drei unteren Substratschichten 19 bis 21 jeweils mit leitenden Strukturen 22 versehen, welche aus inerten Edelmetallen, z.B. Gold oder Platin bestehen können und die Ableitungen bilden, wobei ihre in Fig. 3 rechts gelegenen Stirnflächen 23 den Flächen 7 im Beispiel der Fig. 1 entsprechen. Diese Stirnflächen 23 sind mit den zugehörigen Stirnflächen 24 der Schichten 18 bis 21 bündig. Die im Bereich der Stirnfläche 23, 24 zwecks Unterbringung der Membrane auf auf einer möglichst kleinen Fläche zusammengeführten Strukturen 22 sind am entgegengesetzten, in Fig. 3 links gelegenen Ende der Schichten 19 bis 21 auseinandergeführt und elektrisch leitend durch Kontaktierungen 25 jeweils mit Anschlußflächen 26 der hier obersten Schicht 18 verbunden, wobei die Anschlußflächen 26 den Anschlüssen 8 der Fig. 1 entsprechen und ebenfalls an eine Rechnerelektronik oder dergleichen angeschlossen werden. Dieser Anschluß ist bevorzugt in Form einer Steckverbindung ausgebildet, so daß schnell und einfach unterschiedliche Sensoren mit der Rechnerelektronik oder dergleichen verbunden werden können (siehe Fig. 9).

Nach dem Laminieren und Einbrennen oder Härten des Sensorkörpers wird die in Fig. 3 rechts dargestellte Seite mechanisch (z.B. Sägen oder Schleifen) entlang der in Fig. 6 eingezeichneten Schnittlinie A-A so bearbeitet, daß dort die leitenden Strukturen 22 mit ihren Stirnflächen 23 an die Oberfläche treten und somit ihr elektrischer Kontakt mit den nachstehend zu erläuternden Membranen 29 möglich ist.

Der Träger 28, z.B. eine Polymerschicht, wird auf die Stirnfläche 24 des Multilayersubstrates aufgegossen oder in anderer Weise fest aufgebracht. Die in Fig. 6 durch Gegenschraffur angedeuteten Membranen 29 sind entweder in dem Träger 28 vor dessen Befestigen an dem Multilayersubstrat bereits enthalten; oder aber sie werden nach dessen Befestigung am Multilayersubstrat in ihn eingebracht. Dazu können im Träger durch elektrische Entladungen, durch Laserstrahlen oder dergleichen Durchbrüche erzeugt werden, in welche die Membranen eingesetzt werden. Insbesondere empfiehlt sich die Einbringung der Membranen in Form von Lösungen, wobei nach Verdunsten des Lösungsmittels die Membranen die gewünschten Eigenschaften aufweisen. Hierdurch wird eine besonders innige und damit den Durchtritt von Feuchtigkeit sperrende Verbindung zwischen dem Membranmaterial und dem Trägermaterial erreicht. Man kann aber auch so vorgehen, daß die Membranen auf mechanischem Wege ( z. B. durch Stanzen) in ihrer Grundform aus einem folienartigen Material herausgearbeitet und durch ein Klebemittel oder ein Lösungsmittel in den Durchbrüchen des Trägers befestigt und feuchtigkeitsdicht verklebt werden. Die Membranen 29 stehen auch in diesem Ausführungsbeispiel in unmittelbar leitendem Kontakt mit den Stirnflächen 23 der Ableitungen 22.

Die Fig. 4 und 5 zeigen eine, z.B. matrixartige, Struktur der Stirnflächen 23, bzw. der Membranen 29. Dies heißt, daß eine entsprechend große Anzahl leitfähiger Kontaktstellen (Membranen) auf einer sehr kleinen Fläche (z.B. 4 Membrane auf einer Fläche von 1 x 1 mm²) untergebracht werden kann. Statt dieser matrixartigen Anordnung beispielsweise in drei Reihen zu je vier Membranen könnten die Membranen auch anders, z.B. in mehreren ineinanderliegenden Kreisen, spiralförmig und dergleichen mehr angeordnet sein. Andererseits können aber die zugehörigen Anschlußflächen 26 an der Schicht 18 auf einer gegenüber der von den Membranen eingenommenen Fläche wesentlich größeren Fläche untergebracht werden. Dies ist besonders im Fall einer lösbaren Steckverbindung (siehe oben) von Vorteil.

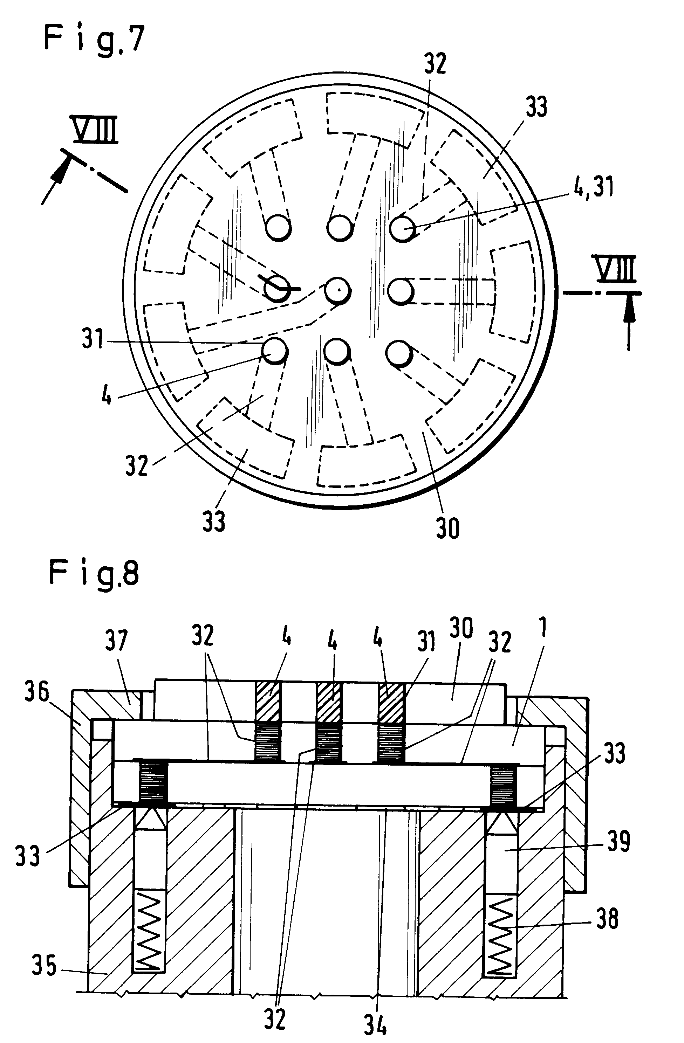

In der Ausführungsform der Fig. 7 und 8 ist ein Träger 30 in Form einer zylindrischen Kreisscheibe vorgesehen, in deren Durchbrüche 31 Membranen 4 in der Ausbildung gemäß Fig. 1 feuchtigkeitsdicht eingelassen sind. Jede Membrane ist unterseitig über eine Ableitung 32 mit einer Kontaktstelle 33 verbunden, die sich am äußeren Umfang der in Fig. 8 dargestellten unteren Stirnfläche 34 des Sensorkörpers 1 befindet. Mit diesem äußeren, die Kontaktstellen 33 aufweisenden Umfang ruht der Sensorkörper 1 auf der oberen Stirnfläche eines zylindrischen Halters 35 (Fig. 8). Dieser Halt wird durch eine Überwurfmutter 36 gesichert, die mit ihrem nach innen weisenden Rand 37 den in Fig. 8 oberen, sowie äußeren Bereich des Sensorkörpers 1 erfaßt und gegen den Halter 35 drückt. Im Halter 35 sind gegen Federwirkung 38 Kontaktstifte 39 gelagert, welche die Kontaktstellen oder -flächen 33 kontaktieren. Der Sensorkörper 1 kann laminiert sein (siehen das vorhergehende Ausführungsbeispiel). Diese Ausführungsform der Erfindung ist mit relativ geringen Kosten herstellbar. Die Überwurfmutter 36, 37 schafft eine sehr gute Abdichtung. Im dargestellten Ausführungsbeispiel der Fig. 7 und 8 ist der z.B. aus einem Polymer bestehende Träger 30 mit dem z.B. aus einer Aluminiumoxyd-Keramikfolie bestehende Sensorkörper 1 fest verbunden. Man kann aber auch Träger und Sensorkörper 1 aus der gleichen Aluminiumoxyd-Keramikfolie herstellen und miteinander durch Erhitzen zu einer Einheit verschmelzen (in der Zeichnung nicht dargestellt).

Grundsätzlich gilt, daß der Sensor im Fall eines Einsatzes für medizinische Zwecke aus sterilisierbarem Material besteht.

Fig. 9 zeigt im Prinzip die Rechnereleketronik 40 mit einer flexiblen, beweglichen Zuleitung 41 zu einer Haltevorrichtung 42, die in ihrer Form selber als Handhabe ausgebildet sein kann, oder mit einem Haltegriff versehen ist. An das äußere Ende 43 der Haltevorrichtung 42 ist der hier mit 44 bezifferte Sensor lösbar aufgesteckt. Diese leichte und schnelle Auswechselmöglichkeit der Sensoren ist insbesondere bei Operationen von wesentlicher Bedeutung. Dies kann z.B. beim Ausführungsbeispiel der Fig. 3 so geschehen, daß die Anschlüsse 26 in dieser Zeichnung nach links herausgeführt sind und in Steckern enden, welche in entsprechende Steckeröffnungen des Endes 43 passen. Auch könnte man den Halter 35 gemäß Fig. 7, 8 an seinem Ende mit entsprechenden Steckern ausbilden, welche an die Kontaktstifte 39 angeschlossen sind.

Ferner kann im Sensor ein Temperaturfühler integriert bzw. eine Temperaturmeßeinrichtung vorgesehen sein. Die gemessene Temperatur wird dann in den Rechner 9 oder dergleichen zur automatischen Temperaturkorrektur eingegeben.

Zwischen den abgehenden Kontakten 8, 11, 15, 26, 33 einerseits und der Rechnerelektronik 9 oder dergleichen andererseits kann ein sogenannter Impedanzwandler vorgesehen sein. Dieser hat die Aufgabe, die durch das Meßprinzip der ionenselektiven Messung entstehenden, sehr hochohmigen Signale in niederohmige Signale umzuwandeln, die dann über eine Leitung weitergegeben werden können. Diese Hochohmigkeit bedingt eine entsprechende Störanfälligkeit bei Messungen. Um eine ausreichende Abschirmung vor Umwelteinflüssen zu erreichen, ist daher möglichst unmittelbar am Sensor die o.g. Impedanzwandlung vorzusehen. Nur somit ist die z.B. für Operationen notwendige Kabellänge von kleiner als 5 m erreichbar. An diesem Impedanzwandler kann sich dann ein Wandler zur Umwandlung der vom Impedanzwandler abgegebenen analogen niederohmigen Signale in digitale Signale für den Rechner anschließen.

Der Impedanzwandler, der Analog-Digital-Wandler und zumindest ein Teil der Rechnerelektronik sind vorteilhafterweise im Handgriff 42 vorgesehen. Dies ergibt den Vorteil sehr kurzer Leitungswege bzw. direkter Anschlußverbindungen vom Sensor zum Impedanzwandler und von diesem zum Analog-Digital-Wandler, sowie von diesem zur Rechnerelektronik.

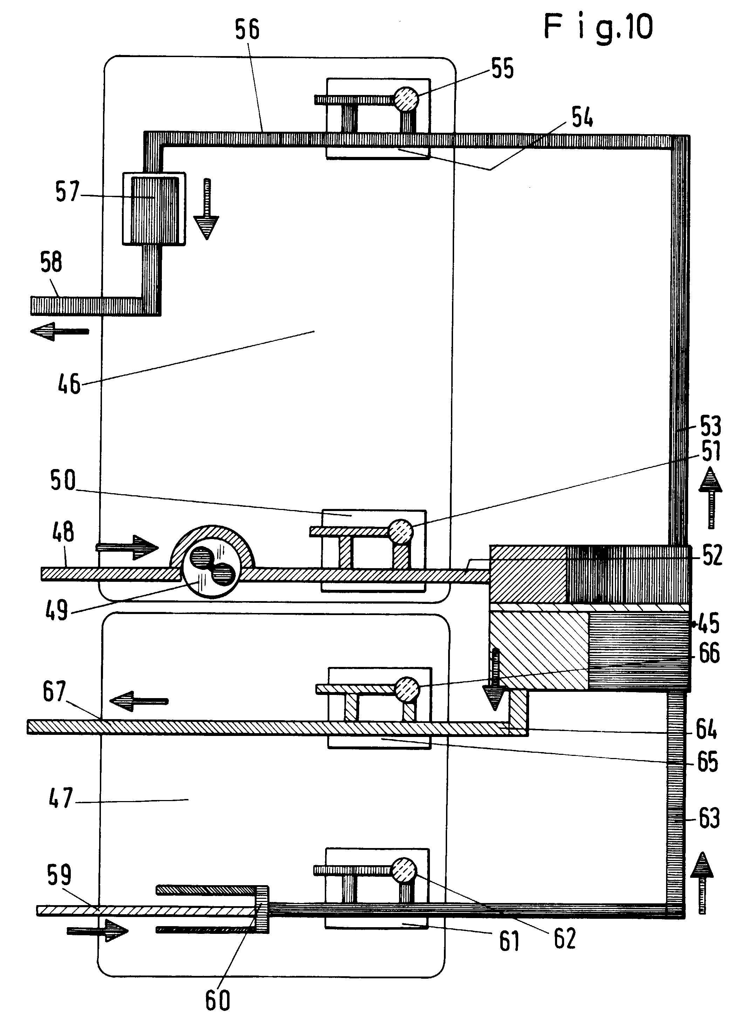

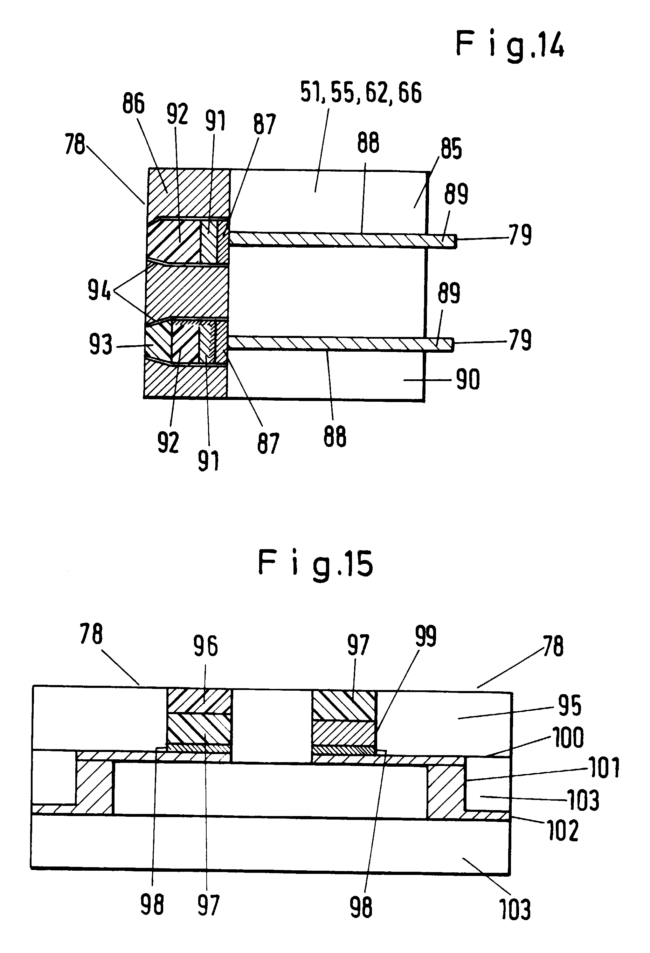

Fig. 10 zeigt eine Dialyseanordnung, bestehend aus dem Dialysator (sogenannte ("künstliche Niere") 45, dem Blutmonitor 46 und dem Dialysatmonitor 47, sowie den nachstehend im einzelnen erläuterten Leitungen, Sensoren und weiteren Bauteilen. Hinsichtlich des Aufbaues und Ausgestaltung der Sensoren wird auf die vorstehenden Ausführungen, aber auch auf die Ausführungsbeispiele gemäß den Fig. 14 und 15 verwiesen. Das Blut wird durch die Leitung 48 vom Patienten her mit Hilfe einer Blutpumpe 49 einer dritten Meßzelle 50 mit dem dritten Sensor 51 und von dort über eine Leitung 52 dem Dialysator zugeführt. Nach der sogenannten Blutwäsche wird das gereinigte Blut über die Leitung 53 einer ersten Meßzelle 54 und damit dem ersten Sensor 55 zugeführt. Von dort gelangt das Blut über eine Leitung 56 und eine Luftfalle 57 zur Ausgangsleitung 58 und von dieser wieder in den Körper des Patienten zurück. Die Dialysatflüssigkeit wird durch die Leitung 59 und über ein Dialysatmischsystem 60 einer zweiten Meßzelle 61 mit dem zweiten Sensor 62 und von dort über eine Leitung 63 dem Dialysator 45 zugeführt. Dies aus dem Dialysator abfließende Dialysatflüssigkeit gelangt durch die Leitung 64 zu einer vierten Meßzelle 65 mit dem vierten Sensor 66 und von da zur Abflußleitung 67. Wie bereits eingangs dargelegt, wird zwischen dem ersten Sensor 55 und dem dritten Sensor 51 die sogenannte Reinigung des Blutes überwacht, während der zweite Sensor 62 zur Kontrolle des Dialysatmischsystemes und der vierte Sensor 66 zur quantitativen Kontrolle des Elektrolytverlustes während der Dialyse dient. Zumindest sind der erste Sensor 55 und der zweite Sensor 62 erforderlich, um das Dialysatmischsystem aufgrund der Zusammensetzung des den Dialysator 45 verlassenden Blutes mit Hilfe des ersten Sensors 55 zu kontrollieren, bzw. zu regeln, wobei die Zusammensetzung des Blutes nach Verlassen des Dialysators, d.h. am Sensor 55 die Regelgröße für die Einstellung des Dialysatmischsystemes bildet. Es kann ein rein schematisch dargestellter Rechner 68 vorgesehen sein, der über nicht gezeichnete Leitungen mit den Ausgängen der o.g. Sensoren verbunden ist, deren Meßergebnisse auswertet und das Dialysatmischsystem entsprechend regelt. Hierzu kann im Rechner 68 ein Sollwert der Zusammensetzung des Blutes eingestellt werden.

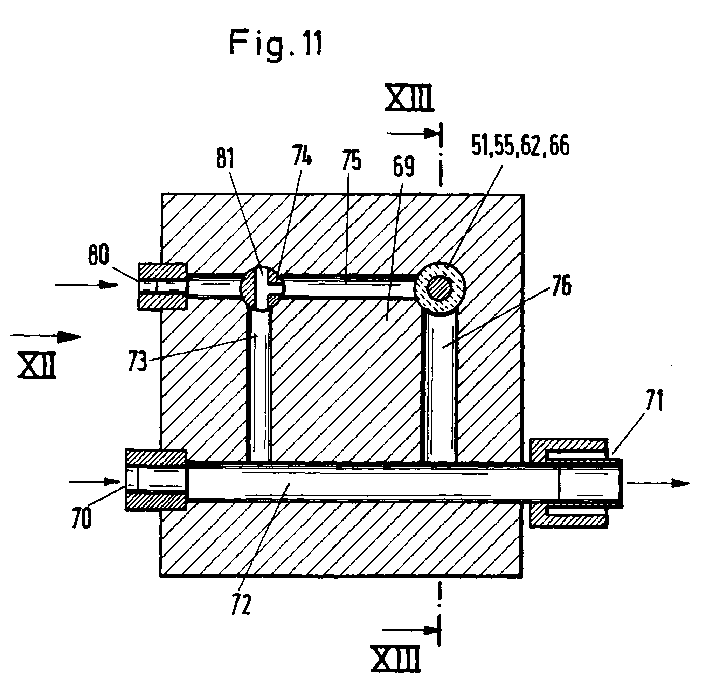

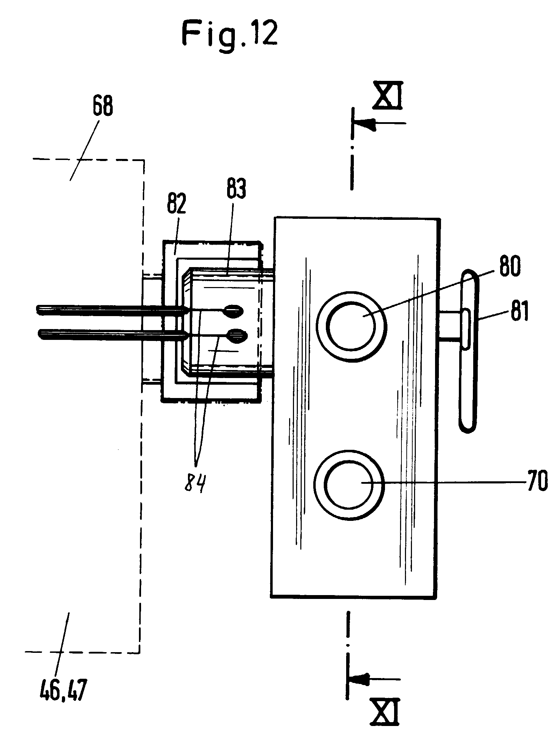

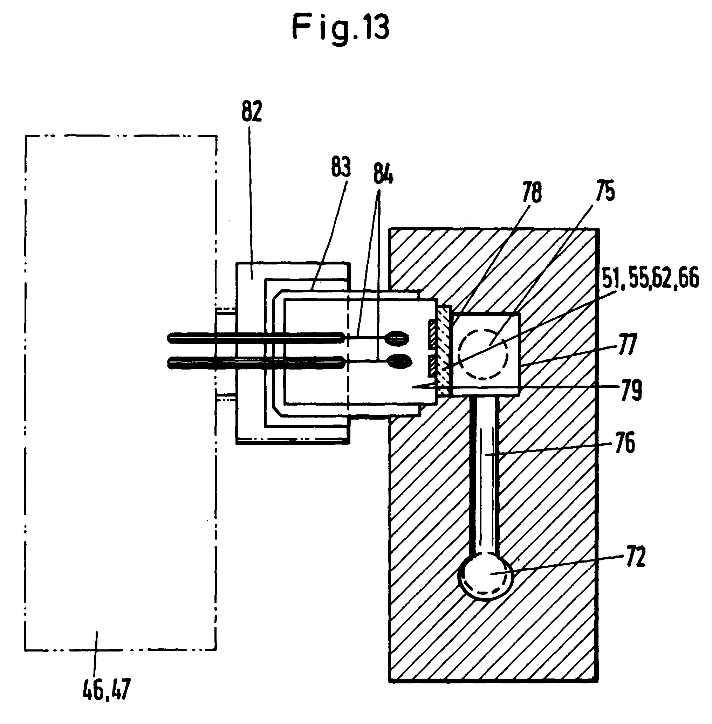

Der Aufbau der o.g. Meßzellen ist den Fig. 11 bis 13 zu entnehmen. Innerhalb des Meßzellenkörpers 69 ist der jeweilige Sensor 51, 55, 62, 66 untergebracht und befestigt. Beim Anschluß 70 wird die zu messende Flüssigkeit zugeführt und beim Anschluß 71 abgeführt. Im Nebenschluß zu dem Verbindungskanal 72 zwischen den beiden Anschlüssen 70 und 71 wird über einen Kanal 73 und einen Dreiwegehahn 74 sowie einen weiteren Kanal 75 die zu messende Flüssigkeit am Sensor vorbeigeführt (siehe hierzu auch Fig. 13), dort gemessen und über den Kanal 76 dem Anschluß 71 zugeleitet. Zu diesem Zweck ist neben dem Sensor eine Meßkammer 77 (Fig. 13) vorgesehen, in welche die Kanäle 75, 76 münden. Die Kontakt- oder Meßseite des Sensors ist mit 78 und dessen Anschlußseite mit 79 beziffert.

Wird der Dreiwegehahn aus der in Fig. 11 gezeichneten Position um 90° entgegen dem Uhrzeigersinn gedreht, so wird der Zufluß der zu messenden Flüssigkeit (Blut oder Dialyseflüssigkeit) vom Kanal 73 her gestoppt und der Zufluß einer Kalibrier- oder Eichflüssigkeit vom Anschluß 80 über einen Kanal 81 zum Kanal 75 und damit zum Sensor ermöglicht. Die Eichflüssigkeit ist, soweit sie ins Blut gelangt, blutverträglich und steril, sowie eine isotonische Lösung. Es versteht sich, daß die gesamte Meßzelle mit Sensor steril gehalten sein muß.

Die Meßzellen sind am Blutmonitor 46 und Dialysemonitor 47 befestigt, wobei diese Befestigungen bevorzugt lösbar sind. Hierzu können Überwurfmuttern 82 dienen. die je mit einem Innengewinde über ein Außengewinde von Stutzen 83 geschraubt werden, welche sich an der jeweiligen Meßzelle befinden. Hiermit werden zugleich Federkontakte 84 des jeweiligen Monitors mit der Anschlußseite 79 des jeweiligen Sensors in einen elektrischen Berührungskontakt gebracht. Die Federkontakte stellen die Kontaktverbindungen zwischen den Sensoren und den jeweiligen Rechen- bzw. Regelteilen der Monitoren oder dem Rechner 68 her.

Fig. 14 zeigt einen der Sensoren 51, 55, 62, 66 mit Sensorkörper 85, Träger 86 für die noch zu erläuternde Membrane, Ableitungen 87 und deren Verbindungen 88 zu Anschlüssen 89. Es können mehrere Membrangruppen vorgesehen sein. Eine der Membrangruppen dient zusammen mit der zugehörigen Ableitung im vorliegenden Ausführungsbeispiel als Bezugselektrode und die andere Membrangruppe bzw. die anderen Membrangruppen zusammen mit den zugehörigen Ableitungen als Meßelektrode, bzw. Meßelektroden. Die Kontakt- oder Meßseite ist auch hier mit 78 und die Anschlußseite mit 79 beziffert. Der Träger 86 besteht aus einem isolierenden Werkstoff, z.B. einem Polymer. Der Sensorkörper 90 ist so aufgebaut und aus einem solchen Werkstoff, daß zwischen den Ableitungen 87, sowie deren Verbindungen 88 kein elektrischer Kontakt hergestellt werden kann. Im einzelnen wird auf die obigen Ausführungen verwiesen.

Es sind hier mehrere Membrane, in diesem Ausführungsbeispiel unten drei Membranen 91, 92 und 93 als Gruppe übereinander vorgesehen, wobei die in der Aussparung 94 in Fig. 14 am weitesten rechts gezeichnete Membrane 91 in Berührungskontakt mit der jeweiligen Ableitung 87 steht und andererseits auch Berührungskontakt mit der links von ihr befindlichen Membrane 92 hat, die ihrerseits in Berührungskontakt mit der weiteren Membrane 93 ist, welche die äußere Kontaktfläche dieser Membrananordnung bildet. In Fig. 14 oben sind nur die beiden Membranen 91, 92 vorgesehen. Die einzelnen Membrane einer solchen säulenartigen Membrananordnung sind aufeinander abgestimmt. Eine der Membranen ist die eigentliche Meßmembrane und eine andere, bzw. die beiden anderen Membranen einer Gruppe dienen der Entfernung oder Absorbierung von Störionen.

Zur Herstellung einer solchen Membrananordnung kann man so verfahren, daß man die einzelnen Membrane durch Auflösen mittels eines Lösungsmittels oder durch einen Schmelzvorgang zumindest in ihren Randbereichen innerhalb der Aussparung 94 verflüssigt, wobei nach Verdunsten des jeweiligen Lösungsmittels oder Abkühlung diese Membrane fest und unlösbar mit der Aussparung verbunden ist. Die Aussparung 94 kann in Richtung zur jeweils außen gelegenen, die äußere Kontaktfläche bildenden Membrane 92 bzw. 93 sich leicht konisch verjüngen (in der Zeichnung ist diese Konizität übertrieben stark dargestellt), um dadurch einen besonders festen Halt der einzelnen Membrane innerhalb der Aussparung zu erreichen. Die Membranen können aus einer Folie, z.B. als ionenselektive PVC-Membrane oder auch Elektrolytgel hergestellt sein. Da in jedem Fall die einzelnen Membrane oder Gele aufeinanderliegen, diffundiert die feuchte bzw. flüssige, zu messende Substanz durch die einzelnen Membranen bis zur Ableitung hin durch.

Während die Ausführung nach der vorstehend erläuterten Fig. 14 vom Prinzip her dem Sensoraufbau gemäß Fig. 1 entspricht, zeigt die nachstehend erläuterte Fig. 15 einen Sensoraufbau etwa gemäß Fig. 8. Im Träger 95 sind zwei unterschiedliche Membrangruppen vorgesehen. In der linken Membrangruppe ist bündig mit der Kontakt- oder Meßseite 78 eine chemische Membran 96 zur Störioneliminierung vorgesehen. Darunter befindet sich eine ionenselektive PVC-Membran 97 und darunter zur Kontaktgabe eine Ag-AgCl-Schicht 98. Der Aufbau der rechten Membrangruppe sieht bündig mit der Kontakt- oder Meßseite 78 die ionenselektive PVC-Membran 97 und darunter ein KCl-Gel 99 vor. Darunter befindet sich wiederum die Ag-AgCl-Schicht 98.

Auch in diesem Ausführungsbeispiel stehen die einzelnen Schichten bzw. Membrane 96 bis 99 untereinander in direktem Berührungskontakt. Die anfallenden elektrischen Signale werden über Leiterbahnen 100 und Durchkontaktierungen 101 zu weiteren Leiterbahnen oder Kontaktstellen 102 geleitet. Diese Leiterbahnen können aus Silber(Ag) bestehen. Der Sensorkörper ist mit 103 beziffert. Der Träger 95 kann z.B. ein Polymer sein. Er ist mit dem Sensorkörper 103 fest verbunden, der gemäß diesem Ausführungsbeispiel aus zwei miteinander ebenfalls fest verbundenen Aluminiumoxyd-Keramikfolien bestehen kann. Stattdessen könnte man aber sowohl Träger 95 als auch Sensorkörper 103 aus einer entsprechenden Anzahl von Aluminiumoxyd-Keramikfolien herstellen und diese miteinander verbacken, wobei die Folien ineinander verschmelzen und damit eine Einheit bilden (nicht dargestellt). Somit ist in der letztgenannten Ausführung, aber auch bei der entsprechenden Variante des Ausführungsbeispieles der Fig. 7 und 8, eine in sich einstückige Einheit von Träger und Sensorkörper aus dem jeweils gleichen Material geschaffen.

Alle dargestellten und beschriebenen Merkmale, sowie ihre Kombinationen untereinander sind erfindungswesentlich. Insbesondere können die im Zusammenhang mit einem der Ausführungsbeispiele erläuterten und gegebenenfalls dargestellten Merkmale sinngemäß auch bei einem der anderen Ausführungsbeispiele eingesetzt werden.

## Patentansprüche

1. Sensor zur Messung der Aktivität von Ionen mit Hilfe von ionenselektiven Membranen und zugehörigen Ableitungen wobei zur gleichzeitigen Messung mehrerer Ionenaktivitäten an der Oberfläche von organischen Geweben oder in Flüssigkeiten zumindest zwei Elektroden in einem gemeinsamen isolierenden Träger (3) gehalten sind, jede dieser Elektroden aus einer Ableitung (2) und einer zu ihr in elektrischem Kontakt stehenden Membran (4) aufgebaut ist, daß ferner jede Membran (4) im Träger (3) sowohl elektrisch als auch gegen den Durchtritt von Feuchtigkeit gesichert gehalten ist, daß mindestens eine der Membranen (4) eine ionenselektive Membran ist, daß eine der Elektroden durch eine entsprechende Membran als Bezugselektrode ausgebildet ist, daß alle Elektroden in einem Sensorkörper (1) zusammengefaßt sind, daß der Träger (3) und der Sensorkörper (1) aneinander festgehalten sind und daß der Sensor aus sterilisierbarem Material besteht.

2. Sensor nach Anspruch 1, dadurch gekennzeichnet, daß alle Membranen (4, 16, 29, 91, 92, 93, 96, 97, 99) eines Trägers (3, 13, 28, 30, 86, 95)auf ein bestimmtes Ion eingestellt sind.

3. Sensor nach Anspruch 1, dadurch gekennzeichnet, daß alle Membranen (4, 16, 29, 91, 92, 93, 96, 97, 99) eines Trägers (3, 13, 28, 30, 86, 95) jeweils auf unterschiedliche Ionen eingestellt sind.

4. Sensor nach einem der Ansprüche 1 bis 3, dadurch gekennzeichnet durch die Kombination von Membranen gemäß den Ansprüchen 2 und 3 in einem einzigen Träger.

5. Sensor nach einem der Ansprüche 1 bis 4, dadurch gekennzeichnet, daß der Sensor als bewegliche Einheit ausgebildet ist.

6. Sensor nach Anspruch 5, gekennzeichnet durch eine Haltevorrichtung (42), an der der Sensor (44) angebracht ist, wobei die Haltevorrichtung als Handgriff ausgebildet oder mit einem Handgriff versehen ist.

7. Sensor nach einem der Ansprüche 1 bis 6, dadurch gekennzeichnet, daß zwischen dem jeweiligen Sensorkörper (1, 13, 18 -21, 85, 103) und einer Rechnerelektronik (40, 68) ein Impedanzwandler vorgesehen ist.

8. Sensor nach einem der Ansprüche 1 bis 7, dadurch gekennzeichnet, daß in der Haltevorrichtung (42) zumindest ein Teil der Rechnerelektronik (40, 68) untergebracht ist.

9. Sensor nach Anspruch 7 oder 8, dadurch gekennzeichnet, daß in der Haltevorrichtung (42) der Impedanzwandler untergebracht ist, der einerseits einen unmittelbaren Anschluß an den in die Haltevorrichtung eingesetzten Sensor und ferner einen entsprechenden Anschluß an den in der Haltevorrichtung untergebrachten Teil der Rechnerelektronik besitzt.

10. Sensor nach einem der Ansprüch 1 bis 9, dadurch gekennzeichnet, daß der Sensor gegen einen Sensor mit anderen Membranen austauschbar ist, wobei im Falle des Vorsehens einer Haltevorrichtung (42) die jeweiligen Sensoren (44) daran lösbar zu befestigen sind und eine lösbare Kontaktierung zwischen den Ableitungen des Sensors und entsprechenden Anschlüssen der Haltevorrichtung besteht.

11. Sensor nach einem der Ansprüche 1 bis 10, dadurch gekennzeichnet, daß die Membranen (4, 16,29, 91, 92, 93, 96, 97, 99) räumlich dicht beieinander auf einer möglichst kleinen Fläche zur Messung an der Oberfläche von organischen Geweben angeordnet sind.

12. Sensor nach einem der Ansprüche 1 bis 11, dadurch gekennzeichnet, daß der Träger (3, 13, 28, 30, 86, 95) aus Polymer besteht, bzw. eine Polymerschicht darstellt.

13. Sensor nach einem der Ansprüche 1 bis 12, dadurch gekennzeichnet, daß ein Sensorkörper aus einer Reihe von miteinander fest verbundenen Schichten (18 -21) vorgesehen ist, daß die einzelnen Schichten voneinander und nach außen isoliert leitende Strukturen als Ableitungen (22) aufweisen, welche an einer Stirnfläche (24) des Sensorkörpers Kontaktstirnflächen (23) bilden und daß jede dieser Ableitungen mit einem zugehörigen Anschlußkontakt oder einer Anschlußfläche (26) des Sensorkörpers, bevorzugt auf dessen oberster Schicht (18), elektrisch leitend verbunden ist.

14. Sensor nach Anspruch 13, dadurch gekennzeichnet, daß der Sensorkörper als Multilayersubstrat ausgeführt ist und aus schichtweise übereinander liegenden und miteinander verbundenen, mit den Ableitungen (22) versehenen Keramikfolien oder polymeren Folien besteht.

15. Sensor nach Anspruch 13 oder 14, dadurch gekennzeichnet, daß die Ableitungen (22) der betreffenden Schichten (19 bis 21) des Multilayersubstrates über Durchkontaktierungen (25) elektrisch mit den Anschlußkontakten oder Anschlußflächen (26) der weiteren Schicht (18) verbunden sind.

16. Sensor nach Anspruch 15, dadurch gekennzeichnet, daß die Anschlußkontakte oder Anschlußflächen (26) als Steckverbindungen zur lösbaren und zugleich kontaktgebenden Anbringung des Sensors an einer Haltevorrichtung (42) ausgebildet sind.

17. Sensor nach einem der Ansprüche 1 bis 12, dadurch gekennzeichnet, daß der Sensorkörper (1) als zylindrische Scheibe ausgebildet und auf einem Halter (35), z. B. mit Hilfe einer Überwurfmutter (36), fest aber lösbar befestigt ist.

18. Sensor nach Anspruch 17, dadurch gekennzeichnet, daß Ableitungen (32) des Sensorkörpers (1) in Kontaktstellen (33) enden, die auf einer Stirnfläche des Halters( (35) aufliegen, wobei die Stirnfläche entsprechende Gegenkontakte (39) aufweist.

19. Sensor nach einem der Ansprüch 1 bis 18, dadurch gekennzeichnet, daß der Träger (3, 28) mit den Membranen (4, 29) auf einer von dem jeweiligen Sensorkörper (1, 18 bis 21) gebildeten Stirnfläche (1', 24) aufliegt, wobei die Kontaktflächen (7, 23) der Ableitungen (2, 22, 32) sich in den Stirnflächen (1', 24) befinden und dort in Kontakt mit entsprechenden Gegenflächen der Membranen (4, 29) stehen.

20. Sensor nach einem der Ansprüche 1 bis 19, dadurch gekennzeichnet, daß ein Temperaturfühler integriert oder eine Temperaturmeßeinrichtung vorgesehen und mit der jeweiligen Rechnerelektronik (9) oder der gleichen verbunden ist.

21. Sensor nach einem der Ansprüche 1 bis 12, dadurch gekennzeichnet, daß als Ableitungen die Enden voneinander isolierter und zu einem Bündel zusammengefaßter Drähte (14) dienen, wobei dieses Bündel durch eine Vergußmasse (13) zusammengehalten ist, die den Sensorkörper und damit einstückig den Träger für die Membranen (16) bilden.

22. Sensor nach einem der Ansprüche 1 bis 21, dadurch gekennzeichnet, daß zwei oder mehrere Membrane (91 bis 93) in einem Träger übereinander vorgesehen sind und daß jede der Membrane mit der benachbarten Membrane in Berührungskontakt steht.

23. Sensor nach Anspruch 22, gekennzeichnet durch Membrane in Folienform, z.B. PVC-Folien.

24. Sensor nach Anspruch 22, gekennzeichnet durch Membrane in Gelform.

25. Sensor nach einem der Ansprüche 22 bis 24, gekennzeichnet durch Aussparungen (94) im Träger (86) zur Aufnahme der Membrane, wobei sich die Aussparungen (94) von ihrer der Ableitung (87) gegenüberliegenden Seite zur Meßseite (78) hin leicht konisch verjüngen.

26. Verwendung von Sensoren nach einem der Ansprüche 1 bis 25 in einer Anordnung zur Hämodialyse, wobei zumindest zwei Sensoren (55, 62) vorgesehen sind, von denen der eine erste Sensor (55) in einem vom Blut des Patienten durchflossenen Kreislauf an einer dem Dialysator (45) in Richtung dieses Kreislaufes nachgeordneten Stelle und der andere zweite Sensor (62) im Verlauf der Dialyseflüssigkeit in deren Fließrichtung an einer vor dem Dialysator gelegenen Stelle zwecks ionenselektiver Messung des Blutes und der Dialyseflüssigkeit eingeschaltet sind und daß die Meßergebnisse beider Sensoren (55, 62) zwecks Regelung der Zusammensetzung der Dialyseflüssigkeit einer Regeleinrichtung zugeführt werden, wobei der Regeleinrichtung ein Vergleichs-Sollwert des gereinigten Blutes zur Verfügung steht.

27. Verwendung nach Anspruch 26, dadurch gekennzeichnet, daß für die ionenselektive Messung des Blutes ein weiterer dritter Sensor (51) vorgesehen ist, der sich in Richtung des Kreislaufes des Blutes vor dem Dialysator (45) befindet und daß dessen Meßdaten ebenfalls der Regeleinrichtung zugeführt werden.

28. Verwendung nach Anspruch 26 oder 27, dadurch gekennzeichnet, daß in den Verlauf der Dialyseflüssigkeit ein weiterer vierter Sensor (66) eingeschaltet ist, der sich in der Fließrichtung der Dialyseflüssigkeit betrachtet nach dem Dialysator (45) befindet und dessen Meßdaten ebenfalls der Regeleinrichtung zugeführt werden.

29. Verwendung nach einem der Ansprüche 26 bis 28, dadurch gekennzeichnet, daß jeder Sensor (51, 55, 62, 66) mit seinem Sensorkörper (85) in einer Meßzelle (50, 54, 61, 65) untergebracht ist, die Anschlußstellen (70, 71) für den Zu- und Ablauf der jeweiligen Flüssigkeit und Kanäle (72) zwischen diesen Anschlußstellen, sowie Kanäle (74, 75, 76) von diesen Anschlußstellen zum Sensor aufweist.

30. Verwendung nach Anspruch 29, dadurch gekennzeichnet, daß die Meßzelle (50, 54, 62, 65) eine weitere Anschlußstelle (80) für den Anschluß und Zufuhr einer Eichflüssigkeit und einen Kanal (81) von dieser Anschlußstelle zu einem Dreiwegehahn (74) aufweist, daß vom Dreiwegehahn ein Kanal (73) zur Anschlußstelle (70) für die Flüssigkeitszufuhr führt und daß der Dreiwegehahn wahlweise in eine Stellung zur Verbindung der Anschlußstelle für die Eichflüssigkeit mit dem Sensor oder in eine Stellung für eine Verbindung der Anschlußstelle für die Flüssigkeitszufuhr mit dem Sensor bringbar ist.

31. Verwendung nach Anspruch 29 oder 30, dadurch gekennzeichnet, daß die Meßzellen am Blutmonitor (46) und am Dialysemonitor (47) einer Dialyseanordnung befestigbar sind, welche aus diesen beiden Monitoren und dem Dialysator (45) besteht.

32. Verwendung nach Anspruch 31, dadurch gekennzeichnet, daß als Befestigungsmittel eine Überwurfmutter (82) dient, wobei mit dem Anschrauben der Überwurfmutter die elektrischen Kontaktverbindungen zwischen den Ableitungen (87, 89) des Sensors und entsprechenden elektrischen Gegenkontakten (84) des jeweiligen Rechners bzw. der Regeleinrichtung (68) hergestellt wird.

33. Verwendung nach Anspruch 32, dadurch gekennzeichnet, daß die elektrischen Gegenkontakte als federnde Kontakte (84) ausgebildet sind.

## Claims

1. Sensor for measuring the activity of ions using ion-selective membranes and the appropriate leads whereby for the simultaneous measurement of ion activity in a number of places on the surface of organic tissues or in liquids at least two electrodes are contained in a common, insulating carrier (3), each of the electrodes consisting of a lead (2) and a membrane (4) which is in electrical contact with it, further that each membrane (4) in the carrier (3) is insulated both electrically and against moisture penetration, that at least one of the membranes (4) is an ion-selective membrane, that one of the electrodes has been made the reference electrode by an appropriate membrane, that all the electrodes are collected together in a sensor body (1), that the carrier (3) and the sensor body (1) are joined together and that the sensor is made of sterilisable material.

2. A sensor according to claim 1, wherein each of said membranes (4, 16, 29, 91, 92, 93, 96, 97, 99) is adjusted in the carrier ( 3, 13, 28, 30, 86, 95) to a selected ion for measuring the activity of the selected ion.

3. A sensor according to claim 1, wherein each of said membranes (4, 16, 29, 91, 92, 93, 96, 97, 99) is adjusted in the carrier ( 3, 13, 28, 30, 86, 95) to a different ion for sensing the different ion.

4. A sensor according to claims 1 to 3, with a combination according to claims 2 to 3 of in one said carrier.

5. A sensor according to claims 1 to 4, wherein said sensor body are portable as a single unit.

6. A sensor according to claim 5, wherein said sensor (44) is held in holder, form as a handheldprobe or a unit with a handle.

7. A sensor according to claims 1 to 6, wherein between the said sensorbody (1, 13, 18 - 21, 85, 103) and acomputer system (40, 68) an impedance converter is connected.

8. A sensor according to claims 1 to 7, including a holding device (42) at least one part of said computer electronic (40, 68) is integrated.

9. A sensor according to claim 7 or 8, inclucding said holding device (42) the impedance converter is integrated with a diriect connetion of said sensor to the holding device and a special conector for the computer means.

10. A sensor according to claims 1 to 9, wherein said sensor can be interchanged by a sensor with different membranes, whereby in case of using said holding device (42) the used sensor (44) can be interchanged direct with the holding device or there is a seperable connection between the leads of the sensors and the connections of the holding device.

11. A sensor according to claims 1 to 10, wherein said membranes (4, 16, 29, 91, 92, 93, 96, 97, 99) are as close as possible togeher an a area as smal as possible, to measure on surfaces of organ tissues.

12. A sensor according to claims 1 to 11, wherein said carrier ( 3, 13, 28, 30, 86, 95) comprises polymer.

13. A sensor according to claims 1 to 11, wherein said body comprises a plurality of layers securely connected to each other (18 - 21), a plurality of conductive structures (22) on each layer forming said shunts, each shunt extending to a common contact surface (24) of said body, each of said membranes being connected to one of said shunts at said common contact surface (26), an uppermost one of said layers carrying connecting surfaces each connected to one of said conductive structures for establishing connection with each electrode.

14. A sensor according to claim 13, wherein said carrier is formed as a multi layer substrate and is made of layers of ceramic foils or polymer foils with conductive structures (22).

15. A sensor according to claim 13 or 14, wherein the conductive structures (22) of each layer (19 - 21) of the multi layer substrate is in electrical contact with the outer connecting surfaces (26) by shunts (25) to the next layer (18).

16. A sensor according to claim 15, wherein the connecting surfaces (26) are built up as plug connector to replace the sensor and give the sensor electrical contact with the holding device (42).

17. A sensor according to claims 1 to 12, wherein said sensor body (1) is in the form of a cylindrical disk, a holder for receiving said cylindrical disk and a retaining mean engaged with said holder (35) for fixing said cylindrical disk on said holder.

18. A sensor according to claims 17, wherein wherein said conductin surfaces (32) of the sensor (1) has a contact surface (33), said shunts having contact points ending at said contact surface of said holder (35), and reciprocal contacts movably mounted in said holder for engaging each of said contact points (39) of said shunts.

19. A sensor according to claims 1 to 18, wherein said holder (3, 28) with the membranes (4,29) has a contact surface (1', 24) with the sensorbody (1, 18 bis 21), said shunts (2, 22, 32) having contact points (7, 23) ending at said contact surface (1', 24) of said holder, having contact with the reciprocal contacts surfaces of the membranes (4, 29).

20. A sensor according to claims 1 to 19, including a temperature sensing device integrally formed in said carrier and adapted for connection to computing means (9).

21. A sensor according to claims 1 to 12, wherein said shunts each comprise a wire extending through said body, said body and carrier being formed as a single piece from a casting compound cast around said wires (14), said wires being spaced from each other in said casting compound forming the sensorbody as one carrier for the membranes (16).

22. A sensor according to claim 1 to 21, wherein at least two or more of said membranes (91 - 93) comprises a plurality of adjacent contacting membrane layers in one carrier.

23. A sensor according to claim 22, wherein at least one of said membrane layers is made of foil.

24. A sensor according to claim 22, wherein at least one of said membrane layers is made of gel.

25. A sensor according to claim 22 to 24, including a cut-out portion (94) in said carrier (86) having a slightly conical shape, said plurality of membrane layers being disposed in said cut-out.

26. Use according to claim 1 to 5, in combination with a hemodialyis system, that at least two sensors (55, 62), one of said sensors (55) disposed in said blood circulation path downstream of said dialyzer (45), the otherone of said sensors (62) disposed in said dialysis fluid circulation path upstream of said dialyzer and a control device for providing a reference desired value for the condition of blood at said first electrode connected to said first and second electrodes for controlling said dialysis fluid.

27. Use according to claim 26, including a third one of said sensors (51) in said blood circulation path upstream of said dialyzer (45), said third sensors being connected to said control device for measuring the difference in condition of said blood upstream and downstream of said dialyzer.

28. Use according to claim 26 or 27, including a fourth one of said sensors (66) in said dialysis fluid circulation path downstream of said dialyzer (45) and connected to said control device for measuring differences in condition between said dialysis fluid upstream and downstream of said dialyzer.

29. Use according to claim 31 or 27, including a measuring cell (50, 54, 61. 65) for each of said first, second, third and fourth sensors (51, 55, 62, 66), each measuring cell having connection points (70, 71) for the inflow and outflow of fluid, and ducts (72) interconnecting said connection points, said sensors being on one of said ducts (74, 75, 76).

30. Use according to claim 29, wherein said measuring cell (50, 54, 62, 65) includes a calibration fluid connection point (80) and a calibration fluid duct (81) extending to said duct in which said electrode is disposed, and a three-way stop cock (74) in a junction between said calibration fluid duct and said duct in which said electrode is disposed for selectively applying fluid to said electrode either from said calibration fluid duct or one of said first mentioned connection points.

31. Use according to claim 29 or 30, including a blood monitor (46) said first sensor and a dialysis monitor (47) and the dialyser (45) for receiving at least said second sensor.

32. Use according to claim 31, wherein said measuring cell includes a fastening projection (82) for receiving electrical contacts connected to said sensor in said measuring cell, said control device comprising computing means (68) having reciprocal contacts (84) engageable with said electrical contacts (87,89), and a retaining nut screwed onto said fastening projection for holding said reciprocal contacts to said electrical contacts.

33. Use according to claim 32, wherein the electrical reciprocal contacts (84) are formed as spring contacts.

## Revendications

1. Détecteur servant au mesurage de l'activité des ions moyennant des diaphragmes sélecteurs d'ions et des branchements y afférents, deux électrodes au moins étant retenues dans un commun porte-diaphragmes (3) isolant pour permettre le mesurage simultané de plusieures activités d'ions soit à la surface de tissus organiques soit dans des liquides, chacune desdites électrodes étant composée d'un branchement (2) et d'un diaphragme (4) qui est en contact électrique avec ledit branchement, que chaque diaphragme (4) se trouve monté dans ledit porte-diaphragmes (3) et étant muni non seulement d'un coupe-circuit mais aussi d'une isolation empêchant la pénétration de l'humidité, qu'un desdits diaphragmes (4) au moins est un diaphragme sélecteur d'ions, qu'une des électrodes constitue une électrode de référence ou de comparaison moyennant un diaphragme approprié, que toutes les électrodes sont réunies et intégrées dans un corps détecteur (1), que ledit porte-diaphragmes (3) et ledit corps détecteur (1) sont retenus et fixés l'un à l'autre et que ledit détecteur consiste d'un matériel qui est stérilisable.

2. Détecteur selon la revendication 1, caractérisé en ce que la totalité des diaphragmes (4, 16, 29, 91, 92, 93, 96, 97, 99) compris dans un porte-diaphragmes (3, 13, 28, 30, 86, 95) est ajustée à un ion déterminé.

3. Détecteur selon la revendiation 1, caracterisé en ce que tous les diaphragmes (4, 16, 29, 91, 92, 93 , 96, 97, 99) compris dans un porte-diaphragmes (3, 13, 28, 30, 86, 95) sont respectivement ajustés à des ions différents.

4. Détecteur selon l'une quelconque des revendiations 1 à 3, caracterisé par la combinaison de diaphragmes selon les revendications 2 et 3, combinaison qui se trouve intégrée dans un seul porte-diaphragmes.

5. Détecteur selon l'une quelconque des revendications 1 à 4, caractérisé en ce que ledit détecteur est conçu et construit comme une unité mobile.

6. Détecteur selon la revendication 5, caractérisé par un dipositif porteur (42), auquel ledit détecteur (44) est fixé, ledit dispositif porteur étant conçu sous forme de poignée ou étant pourvu d'une poignée.

7. Détecteur selon l'une quelconque des revendications 1 à 6, caracterisé en ce qu'un transformateur d'adaptation d'impédance est prévu entre le corps respectif d'un détecteur (1, 13, 18-21, 85, 103) d'une part et l'électronique d'un ordinateur (40, 68) d'autre part.

8. Détecteur selon l'une quelconque des revendications 1 à 7, caractérisé en ce qu'une partie au moins des éléments électroniques de l'ordinateur (40, 68) se trouve incorporée dans le dispositif porteur (42).

9. Détecteur selon l'une quelconque des revendications 7 ou 8, caractérisé en ce que le transformateur d'adaptation d'impédance est placé à l'intérieur du dispositif porteur (42), ledit transformateur d'adaptation d'impédance étant d'une part pourvu d'un raccordement directe au détecteur qui se trouve placé à l'intérieur dudit dispositif porteur, et d'autre part étant muni d'un raccordement approprié, par lequel il se trouve raccordé à la partie-même des éléments électroniques de l'ordinateur, qui sont installés dans ledit dispositif porteur.

10. Détecteur selon l'une quelconque des revendications 1 à 9, caractérisé en ce que ledit détecteur est interchangeable contre un détecteur muni d'autres diaphragmes différents, et au cas où un dispositif porteur (42) y est prévu, les détecteurs respectifs (44) pouvant y être fixés de façon amovible, et en ce qu'il y existe un contact déconnectable entre les branchements du détecteur et les raccordements correspondants du dispositif porteur.

11. Détecteur selon l'une quelconque des revendications 1 à 10, caractérisé en ce que les diaphragmes (4, 16, 29, 91, 92, 93, 96, 97, 99) sont rangés de façon serrée les uns aux autres sur une superficie qui doit être aussi petite que possible, pour permettre des mesurages à la surface de tissus organiques.

12. Détecteur selon l'une quelconque des revendications 1 à 11, caracterisé en ce que le porte-diaphragmes (3, 13, 28, 30, 86, 95) consiste d'un matériel polymère ou qu'il est constitué par une couche en matériel polymère.

13. Détecteur selon l'une quelconque des revendications 1 à 12, caractérisé en ce qu'un corps détecteur est prévu qui comporte un nombre de couches (18 - 21) qui sont fixées et reliés les unes aux autres, que lesdites couches individuelles sont pourvues des structures conductrices qui sont isolées l'une par rapport à l'autre et vers l'extérieur pour constituter des branchements (22) et formant des faces de contact (23) sur une des faces (24) du corps détecteur, et en ce que chacun de ces branchements se trouve en contact électrique soit avec un contact de jonction y relatif soit avec une face de jonction (26) dudit corps détecteur, et ceci de préférence sur sa couche supérieure (18).

14. Détecteur selon la revendication 13, caractérisé en ce que le corps dudit détecteur est conçu en forme d'un substrat à couches multiples et qu'il consiste de feuilles céramiques ou de feuilles polymères superposées les unes aux autres et liées les unes aux autres, lesdites feuilles étant pourvues des branchements (22).

15. Détecteur selon l'une quelconque des revendications 13 ou 14, caractérisé en ce que c'est moyennant des contacts traversants ou des trous métallisés (25) que les branchements (22) des couches en question (19 à 21) du substrat à couches multiples sont en connexion électrique avec les contacts de jonction ou les faces de jonction (26) de l'autre couche (18).

16. Détecteur selon la revendication 15, caractérisé en ce que les contacts de jonction ou les faces de jonction (26) sont conçus en forme de connecteurs à fiches permettant le montage détachable du détecteur à un dispositif porteur (42) et en même temps assurant la mise en contact électrique entre ledit détecteur et ledit dispositif porteur.

17. Détecteur selon l'une quelconque des revendications 1 à 12, caractérisé en ce que le corps du détecteur (1) est conçu en forme d'un disque cylindrique et qu'il est monté de façon fixe mais détachable sur un support (35), par ex. moyennant un écrou-chapeau (36).

18. Détecteur selon la revendication 17, caractérisé en ce que des branchements (32) du corps du détecteur (1) se terminent en des éléments de contact (33), qui s'appuyent contre une face du support (35), ladite face étant munie de contre-contacts (39) correspondants.

19. Détecteur selon l'une quelconque des revendications 1 à 18, caractérisé en ce que le porte-diaphragmes (3, 28) s'appuye par ses diaphragmes (4, 29) contre une face (1', 24) qui est constituée par le corps-même du détecteur en question (1, 18 à 21), les surfaces de contact (7, 23) desdits branchements (2, 22, 32) se trouvant dans les faces (1', 24) et y étant en contact avec des contre-surfaces correspondantes des diaphragmes (4, 29).

20. Détecteur selon l'une quelconque des revendications 1 à 19, caractérisé en ce qu'une sonde de température y est intégrée ou qu'un dispositif thermométrique est prévu et raccordé aux éléments électroniques de l'ordinateur (9) respectif ou un autre dispositif semblable.

21. Détecteur selon l'une quelconque des revendications 1 à 12, caractérisé en ce que lesdits branchements sont constitués par les bouts de fils (14) qui sont isolés l'un par rapport à l'autre et qui sont groupés pour former un faisceau, ledit faisceau étant réuni et assemblé par une masse de scellement (13), qui constitue le corps du détecteur et en une seule pièce avec celui-ci, le porte-diaphragmes ou corps porteur pour les diaphragmes (16).

22. Détecteur selon l'une quelconque des revendications 1 à 21, caractérisé en ce que deux ou plusieurs diaphragmes (91 à 93) sont prévus dans un porte-diaphragmes l'un en dessus de l'autre, et que chacun desdits diaphragmes est en contact avec le diaphragme voisin.

23. Détecteur selon la revendication 22, caractérisé par des diaphragmes conçus en forme de feuille, par exemple des feuilles en C.P.V.

24. Détecteur selon la revendication 22, caractérisé par des diaphragmes en forme de gel.

25. Détecteur selon l'une quelconque des revendications 22 à 24, caractérisé par des évidements (94) prévus dans le porte-diaphragmes (86) et servant à recevoir des diaphragmes, lesdits évidements (94) ayant une configuration qui s'effile légèrement en forme conique du côté se trouvant en face du branchement (87) vers le côté de mesurage (78).

26. Utilisation de détecteurs selon l'une quelconque des revendications 1 à 25 dans un arrangement servant aux buts de hémodialyse, au moins deux détecteurs (55, 62) étant prévus dont l'un, c'est-a-dire le premier détecteur (55) est intercalé dans un circuit qui est parcouru par le sang du patient, à un point qui se trouve en aval du dialyseur (45) vu en direction de ce circuit, tandis que l'autre, c'est-à-dire le deuxième détecteur (62) se trouve intercalé dans le cours du liquide de dialyse en direction de son écoulement à un point qui se trouve en amont du dialyseur pour ainsi assurer le mesurage sélectif des ions du sang et du liquide de dialyse, et que les résultats des mesurages des deux détecteurs (55, 62) sont amenés à un dispositif régulateur en vue d'assurer le réglage de la composition du liquide de dialyse, ledit dispositif régulateur disposant et pouvant se servir d'une valeur nominale de référence du sang épuré.

27. Utilisation selon la revendication 26, caractérisée en ce qu'un autre, troisième, détecteur (51) est prévu pour le mesurage sélectif des ions du sang, détecteur qui se trouve en amont du dialyseur (45) vu en direction de la circulation du sang, et en ce que les données mesurées par ledit détecteur sont également amenées audit dispositif régulateur.

28. Utilisation selon la revendication 26 ou 27, caractérisée en ce qu'un autre quatrième détecteur (66) est intercalé dans le cours du liquide de dialyse, détecteur qui se trouve en aval du dialyseur (45) vu en direction d'écoulement du liquide de dialyse, les données mesurées par ledit détecteur étant également amenées audit dispositif régulateur.

29. Utilisation selon l'une quelconque des revendications 26 à 28, caractérisée en ce que chaque détecteur (51, 55, 62, 66) avec son corps détecteur (85) se trouve aménagé dans une unité de mesurage (50, 54, 61, 65) qui est pourvu des raccords (70, 71) permettant l'amenée et l'écoulement du liquide respectif, et des passages (72) entre lesdits raccords, ainsi que des passages (74, 75, 76) passant desdits raccords audit détecteur.

30. Utilisation selon la revendication 29, caractérisée en ce que l'unité de mesurage (50, 54, 62, 65) comporte un autre raccord (80) permettant le raccordement et l'amenée d'un liquide étalon et que ladite unité de mesurage comporte en plus un passage (81) qui mène dudit raccord à un robinet à trois voies (74), qu'un passage (73) mène du robinet à trois voies audit raccord (70) pour assurer l'amenée du liquide, et que ledit robinet à trois voies peut facultativement être ajusté soit en une position où ledit raccord du liquide étalon se trouve relié avec ledit détecteur, soit en une position reliant ledit raccord de manière à ce que ledit liquide soit amené audit détecteur.

31. Utilisation selon l'une quelconque des revendications 29 ou 30, caracterisée en ce que les unités de mesurage peuvent être fixées au moniteur du sang (46) et au moniteur de dialyse (47) d'un équipement de dialyse qui consiste desdits deux moniteurs et du dialyseur (45).

32. Utilisation selon la revendication 31, caractérisée en ce qu'un écrou-chapeau (82) sert de moyen de fixation, le boulonnage et serrage dudit écrou-chapeau établissant les contacts électriques entre les branchements (87, 89) du détecteur et des contre-contacts électriques correspondants (84) de l'ordinateur respectif ou bien du dispositif régulateur (68).

33. Utilisation selon la revendication 32, caractérisée en ce que les contre-contacts électriques sont conçus en forme de contacts élastiques (84).
